# EUROPEAN PATENT APPLICATION

(11) **EP 4 656 210 A1**
(43) Date of publication of application: **03.12.2025**
(21) Application number: 25179657.9
(22) Date of filing: 29.05.2025
(51) Int. Cl.: A61L 24/00

(54) **COMPOSITE HYDROGELS FOR TREATING VASCULAR DEFECTS**

(30) Priority: 30.05.2024 GR 20240100404; 06.05.2025 US 202519200123
(71) Applicant: Covidien LP, Mansfield, MA 02048 (US)
(72) Inventor: Touris, Athanasios, Mansfield, MA02048 (US); Hicks, Imari, Mansfield, 02048 (US)
(74) Representative: Gray, James

(57) **Abstract**

Compositions and methods for treating vascular defects are provided. In some embodiments, a hydrogel composition for treating a vascular defect includes a plurality of silicate nanoparticles, where each silicate nanoparticle includes a cationic portion and an anionic portion, and where the silicate nanoparticles are non-covalently crosslinked with each other via electrostatic interactions between the cationic portions and the anionic portions of the silicate nanoparticles; and a synthetic polymer comprising an anionic functional group, where the synthetic polymer is non-covalently crosslinked with the plurality of silicate nanoparticles via electrostatic interactions between the anionic functional group of the synthetic polymer and the cationic portions of the silicate nanoparticles.

## Description

### CROSS-REFERENCE TO RELATED APPLICATION(S)

The present application claims the benefit of priority to Greek Patent Application No. 20240100404, filed May 30, 2024, which is incorporated by reference herein in its entirety.

### TECHNICAL FIELD

The present technology generally relates to biocompatible materials, and in particular, to composite hydrogels for treating vascular defects.

### BACKGROUND

An intracranial aneurysm is a portion of an intracranial blood vessel that bulges outward from the blood vessel's main channel. This condition often occurs at a portion of a blood vessel that is abnormally weak because of a congenital anomaly, trauma, high blood pressure, or for another reason. Once an intracranial aneurysm forms, there is a significant risk that the aneurysm will eventually rupture and cause a medical emergency with a high risk of mortality due to hemorrhaging. When an unruptured intracranial aneurysm is detected or when a patient survives an initial rupture of an intracranial aneurysm, vascular surgery is often indicated. One conventional type of vascular surgery for treating an intracranial aneurysm includes using a microcatheter to dispose a platinum coil within an interior volume of the aneurysm. Over time, the presence of the coil should induce formation of a thrombus. Ideally, the aneurysm's neck closes at the site of the thrombus and is replaced with new endothelial tissue. Blood then bypasses the aneurysm, thereby reducing the risk of aneurysm rupture (or re-rupture) and associated hemorrhaging. Unfortunately, long-term recanalization (i.e., restoration of blood flow to the interior volume of the aneurysm) after this type of vascular surgery occurs in a number of cases, especially for intracranial aneurysms with relatively wide necks and/or relatively large interior volumes.

Another conventional type of vascular surgery for treating an intracranial aneurysm includes deploying a flow diverter within the associated intracranial blood vessel. The flow diverter is often a mesh tube that causes blood to preferentially flow along a main channel of the blood vessel while blood within the aneurysm stagnates. The stagnant blood within the aneurysm should eventually form a thrombus that leads to closure of the aneurysm's neck and to growth of new endothelial tissue, as with the platinum coil treatment. One significant drawback of flow diverters is that it may take weeks or months to form aneurysmal thrombus and significantly longer for the aneurysm neck to be covered with endothelial cells for full effect. This delay may be unacceptable when risk of aneurysm rupture (or re-rupture) is high. Moreover, flow diverters typically require antiplatelet therapy to prevent a thrombus from forming within the main channel of the blood vessel at the site of the flow diverter. Antiplatelet therapy may be contraindicated shortly after an initial aneurysm rupture has occurred because risk of re-rupture at this time is high and antiplatelet therapy tends to exacerbate intracranial hemorrhaging if re-rupture occurs. For these and other reasons, there is a need for innovation in the treatment of intracranial aneurysms. Given the severity of this condition, innovation in this field has immediate life-saving potential.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1A is a partially schematic view of a treatment system configured in accordance with embodiments of the present technology.
FIG. 1B is an enlarged cross-sectional view of a distal portion of the treatment system of FIG. 1A.
FIGS. 2A-2E illustrate an example method of treating an aneurysm using the treatment system of FIGS. 1A and 1B, in accordance with embodiments of the present technology.
FIG. 3A illustrates a neck cover configured in accordance with embodiments of the present technology.
FIGS. 3B-3D illustrate an example method of treating an aneurysm using the neck cover of FIG. 3A, in accordance with embodiments of the present technology.
FIG. 4 illustrates the chemical structures of poly(acrylic acid), poly(acrylic acid) sodium salt, oxidized poly(vinyl pyrrolidone), and poly(ethylene glycol) diacid, which are representative examples of synthetic polymers that may be used in embodiments of the present technology.
FIGS. 5A-5C are schematic illustrations of a hydrogel having shear-thinning properties, in accordance with embodiments of the present technology.
FIG. 6A is a graph of storage modulus, loss modulus, and complex viscosity of a laponite-poly(acrylic acid) (PAA) hydrogel at 3% strain.
FIG. 6B is a graph illustrating shear-thinning behavior of a laponite-PAA hydrogel.
FIG. 6C is a graph illustrating the linear viscoelastic properties of a laponite-PAA hydrogel at a frequency of 10 rad/sec.
FIG. 6D is a graph illustrating the viscosity of a laponite-PAA hydrogel.
FIG. 7A is a photograph of a laponite-PAA hydrogel being injected from a syringe.
FIG. 7B is a photograph of a laponite-PAA hydrogel after injection onto a surface.
FIG. 7C is a photograph illustrating occlusion of a simulated vessel with a laponite-PAA hydrogel.

### DETAILED DESCRIPTION

The present technology relates to compositions configured for delivery to a treatment site in a patient's body, such as an aneurysm or other vascular defect, and associated methods. In some embodiments, for example, a composition for treating an aneurysm includes a plurality of inorganic nanoparticles that are non-covalently crosslinked with each other, and a synthetic polymer that is non-covalently crosslinked with the inorganic nanoparticles to form a shear-thinning hydrogel. For example, the inorganic nanoparticles can include cationic portions and anionic portions that interact with each other, and the synthetic polymer can include an anionic functional group (e.g., a carboxylic acid group) that interacts with the cationic portions of the inorganic nanoparticles. When shear stress is applied to the hydrogel (e.g., during injection of the hydrogel through a catheter), the non-covalent crosslinks between the inorganic nanoparticles and/or the synthetic polymer can be disrupted, thereby reducing the viscosity of the hydrogel so the hydrogel exhibits liquid-like behavior. When the shear stress is removed (e.g., after the hydrogel has been delivered into the aneurysm), the non-covalent crosslinks between the inorganic nanoparticles and/or synthetic polymer can reform, thereby increasing the viscosity of the hydrogel so the hydrogel behaves as a cohesive solid that occludes the aneurysm without leaking.

The present technology can provide many advantages over conventional approaches for aneurysm treatment. For example, conventional treatment methods typically use either a low viscosity embolic agent that gels or solidifies in situ when exposed to physiological conditions at the treatment site, or separate precursor components that are mixed immediately before delivery to form the final embolic agent. However, these approaches may present challenges with long-term storage stability, require additional process steps, and/or introduce timing complications. For example, if the agent gels too quickly, it may clog the delivery device. If the agent gels too slowly, it may leak out of the treatment site, which can have catastrophic results in certain applications such as the treatment of cerebral aneurysms. In contrast, the compositions of the present technology can form an injectable hydrogel that can be supplied in a ready-to-use form and immediately introduced into a catheter at any desired time during an aneurysm treatment procedure, without the need to carry out any preliminary mixing steps prior to such introduction. This approach can improve the reliability, convenience, and efficacy of the treatment procedure.

Moreover, many conventional embolic agents use covalent crosslinkers to form a gel, which are typically toxic and may result in an inflammatory response and/or other undesirable side effects at the treatment site if they leach out of the gel over time. Covalent crosslinking may also produce relatively stiff gels, which may require very high injection forces for delivery into the aneurysm and/or may require precise timing of the crosslinking reaction to avoid clogging or leaking, as discussed above. In contrast, the present technology provides non-covalently crosslinked hydrogels with shear-thinning properties, thus avoiding the use of toxic covalent crosslinkers while also reducing the injection force needed to deliver the hydrogel and improving the safety margin of the treatment procedure. The compositions herein are also watersoluble and can therefore be formulated without organic solvents that may produce toxicity and/or other undesirable side effects (e.g., dimethyl sulfoxide can cause vasospasms, injection pain, and a garlic-like odor).

Embodiments of the present disclosure will be described more fully hereinafter with reference to the accompanying drawings in which like numerals represent like elements throughout the several figures, and in which example embodiments are shown. Embodiments of the claims may, however, be embodied in many different forms and should not be construed as limited to the embodiments set forth herein. The examples set forth herein are non-limiting examples and are merely examples among other possible examples.

The headings provided herein are for convenience only and do not interpret the scope or meaning of the claimed present technology. Embodiments under any one heading may be used in conjunction with embodiments under any other heading.

### I. Overview of Treatment Systems and Methods

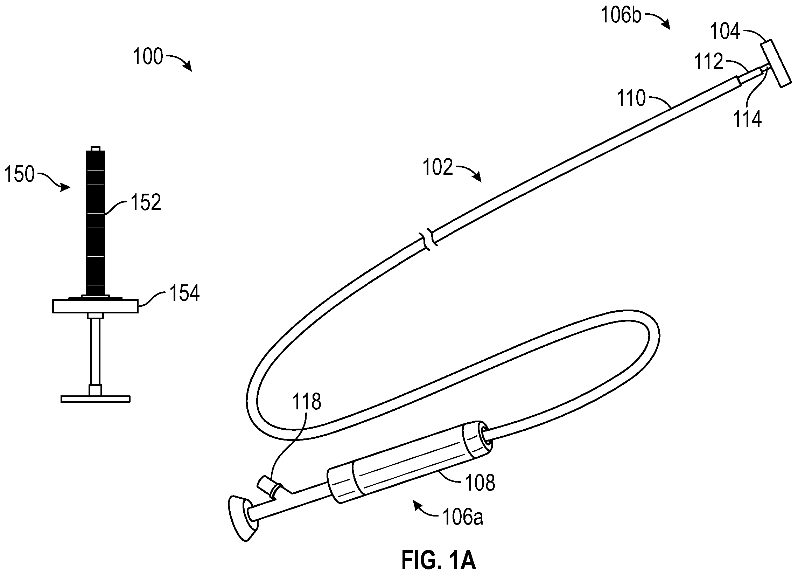

FIG. 1A shows a treatment system 100 ("system 100") configured in accordance with embodiments of the present technology. Although the system 100 is described herein in the context of treating aneurysms such as cerebral aneurysms, this is not intended to be limiting, and the system 100 can also be used in the treatment of other types of vascular defects, and/or in any other application involving delivery of an embolic composition into a space within a patient's body.

As shown in FIG. 1A, the system 100 includes a delivery system 102, a neck cover 104 (also referred to herein as an "occlusive member," "occlusive device," or a "neck protection device"), and an embolic kit 150. The neck cover 104 (shown schematically) is configured to be detachably coupled to the delivery system 102, and the delivery system 102 is configured to intravascularly position the neck cover 104 within an aneurysm. Representative examples of neck covers suitable for use with the system 100 are described in U.S. Patent No. 8,142,456, U.S. Patent No. 9,855,051, U.S. Patent No. 10,327,781, U.S. Patent Application Publication No. 2020/0187953, U.S. Patent Application Publication No. 2021/0128169, and U.S. Patent Application Publication No. 2021/0153872, the disclosures of which are incorporated by reference herein in their entirety.

The embolic kit 150 includes an embolic composition 152 (e.g., a shear-thinning hydrogel as described in Section II below) and an injector 154 configured to be fluidly coupled to a proximal portion of the delivery system 102 for injection of the embolic composition 152 into the aneurysm cavity. The embolic composition 152 can be delivered to a space between the neck cover 104 and the dome of the aneurysm to fill and occlude the aneurysm cavity. The neck cover 104 prevents migration of the embolic composition 152 into the parent vessel, and together the neck cover 104 and embolic composition 152 prevent blood from flowing into the aneurysm. As described in greater detail below, bioabsorption of the embolic composition 152 (in embodiments where the embolic composition 152 is biodegradable) and/or endothelialization of the neck cover 104 may cause the aneurysm wall to fully degrade, leaving behind a successfully remodeled (aneurysm free) region of the blood vessel.

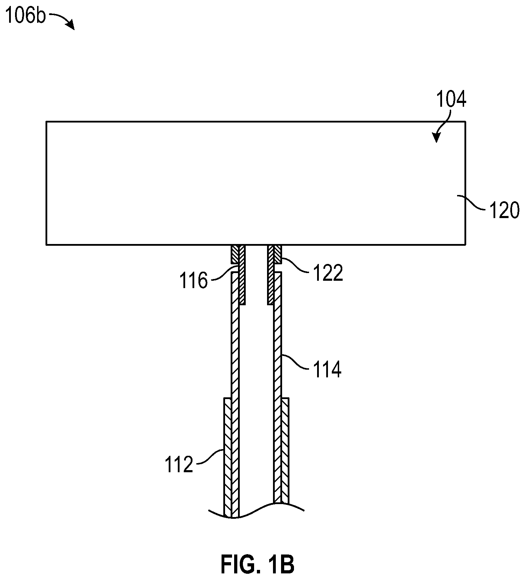

As shown in FIG. 1A, the delivery system 102 has a proximal portion 106a configured to be extracorporeally positioned during treatment and a distal portion 106b configured to be intravascularly positioned at or within an aneurysm. The delivery system 102 may include a handle 108 at the proximal portion 106a and a plurality of elongate shafts extending between the handle 108 and the distal portion 106b. In some embodiments, for example as shown in FIG. 1A, the delivery system 102 may include a first elongate shaft 110 (such as a guide catheter or balloon guide catheter), a second elongate shaft 112 (such as a microcatheter) configured to be slidably disposed within a lumen of the first elongate shaft 110, and a third elongate shaft 114 configured to be slidably disposed within a lumen of the second elongate shaft 112. The delivery system 102 and/or the third elongate shaft 114 is configured to be detachably coupled at its distal end portion to the neck cover 104 via a connector 122 (see FIG. 1B) of the neck cover 104. In some embodiments, the delivery system 102 does not include the first elongate shaft 110.

The second elongate shaft 112 is generally constructed to track over a conventional guidewire in the cervical anatomy and into the cerebral vessels associated with the brain. The second elongate shaft 112 may also be chosen according to several standard designs that are generally available. For example, the second elongate shaft 112 can have a length that is at least 125 cm long, and more particularly may be between about 125 cm and about 175 cm long. The lumen of the second elongate shaft 112 is configured to slidably receive the neck cover 104 in a radially constrained state. The second elongate shaft 112 can have an inner diameter less than or equal to 0.006 inches (0.015 cm), 0.011 inches (0.028 cm), 0.015 inches (0.038 cm), 0.017 inches (0.043 cm), 0.021 inches (0.053 cm), or 0.027 inches (0.069 cm).

The third elongate shaft 114 can be movable within the first and/or second elongate shafts 110, 112 to position the neck cover 104 at a desired location. The third elongate shaft 114 can be sufficiently flexible to enable manipulation (e.g., advancement and/or retraction) of the neck cover 104 through tortuous passages. Tortuous passages can include, for example, catheter lumens, microcatheter lumens, blood vessels, urinary tracts, biliary tracts, and airways. The third elongate shaft 114 can be formed of any material and in any dimensions suitable for the task(s) for which the system 100 is to be employed. In some embodiments, at least the distal portion of the third elongate shaft 114 can comprise a flexible metal hypotube. The hypotube, for example, can be laser cut along all or a portion of its length to impart increased flexibility. In some embodiments, the third elongate shaft 114 can be surrounded over some or all of its length by a lubricious coating, such as polytetrafluoroethylene (PTFE). The third elongate shaft 114 can have an inner diameter less than or equal to 0.006 inches (0.015 cm), 0.011 inches (0.028 cm), 0.015 inches (0.038 cm), 0.017 inches (0.043 cm), 0.021 inches (0.053 cm), or 0.027 inches (0.069 cm)

Referring still to FIGS. 1A and 1B, the embolic composition 152 may be pre-loaded into the injector 154 (as shown) or may be provided separately. The embolic composition 152 can be any material suitable for forming a solid or semi-solid viscoelastic structure (e.g., a hydrogel) that partially or completely occludes the interior cavity of the aneurysm. In some embodiments, the embolic composition 152 is a preformed composition that is ready for use without any mixing of precursor materials. The embolic composition 152 can be sufficiently solid to fill and occlude the aneurysm, without requiring covalent crosslinking reactions to effectively occlude the aneurysm. Additional details of the embolic composition 152 are provided in Section II below.

The injector 154 can be configured to pressurize the embolic composition 152 to a pressure that is sufficiently high to push the embolic composition 152 through the components of the delivery system 102 (e.g., through the lumen of the third elongate shaft 114). As described further in Section II below, the embolic composition 152 can have shear-thinning properties so that relatively low pressures are needed to inject the embolic composition 152 through the delivery system 102, e.g., the embolic composition 152 can be injectable by hand through a standard disposable syringe. In some embodiments, the maximum pressure needed to inject the embolic composition 152 is less than or equal to 5000 psi, 4000 psi, 3000 psi, 2000 psi, 1000 psi, 500 psi, 200 psi, or 100 psi.

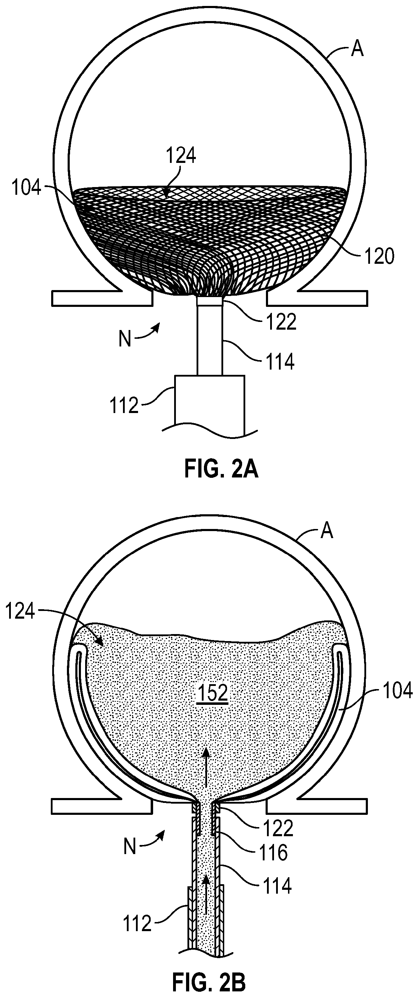

The system 100 can further include a conduit configured to guide the embolic composition 152 delivered from the injector 154 to a space between at least a portion of the neck cover 104 and the aneurysm dome. In some embodiments, the conduit is incorporated into the delivery system 102. For example, as depicted in the enlarged cross-sectional view of the distal portion 106b shown in FIG. 1B, the conduit can comprise a combination of the third elongate shaft 114 and an extension 116 fixed to a distal end portion of the third elongate shaft 114. The extension 116 can be a tubular member that extends distally from the third elongate shaft 114, through the connector 122, and through the neck cover 104, at least when the neck cover 104 is in an expanded state. When the neck cover 104 is collapsed within the lumen of the third elongate shaft 114 during delivery, a portion of the neck cover 104 may extend distally of the extension 116. The length of the extension 116 can be such that, when the distal portion 106b of the delivery system 102 is positioned at the aneurysm with the neck cover 104 in an expanded state (for example, as shown in FIG. 2A), a distal terminus of the extension 116 is even with the distal end of the connector 122, distal of the connector 122 but proximal of a distal end of the neck cover 104, or even with or distal of the distal end of the neck cover 104. It may be beneficial for the extension 116 to be as short as possible to ensure the extension 116 remains sufficiently spaced apart from the fragile aneurysm wall.

In some embodiments, the extension 116 comprises an atraumatic member, such as a soft, flexible coil. In other embodiments, the extension 116 comprises a flexible tube having a continuous sidewall (e.g., not formed of a coiled member). In any case, a distal end portion of the injector 154 can be fluidly coupled to a proximal end portion of the third elongate shaft 114 via a port 118. The port 118 can be located at the proximal portion 106a of the delivery system 102, such as on or proximal to the handle 108. The pressure generated at the injector 154 can cause the embolic composition 152 to flow through the lumen of the third elongate shaft 114, through the lumen of the extension 116, and into the aneurysm cavity. Once the embolic composition 152 has sufficiently filled the aneurysm cavity, the neck cover 104 and extension 116 can be detached via electrolytic detachment that severs a region of the extension 116 exposed between the third elongate shaft 114 and the neck cover 104.

According to several embodiments, the conduit may comprise an additional elongate shaft (not shown). The additional elongate shaft can be delivered to the aneurysm through one or more of the first, second, and/or third elongate shafts 110, 112, 114, or may be delivered separately (e.g., outside of) the delivery system 102. In such embodiments, a proximal end portion of the elongate shaft is configured to be fluidly coupled to the injector 154 via the port 118. Methods for delivering the embolic composition 152 through a separate elongate shaft are discussed below.

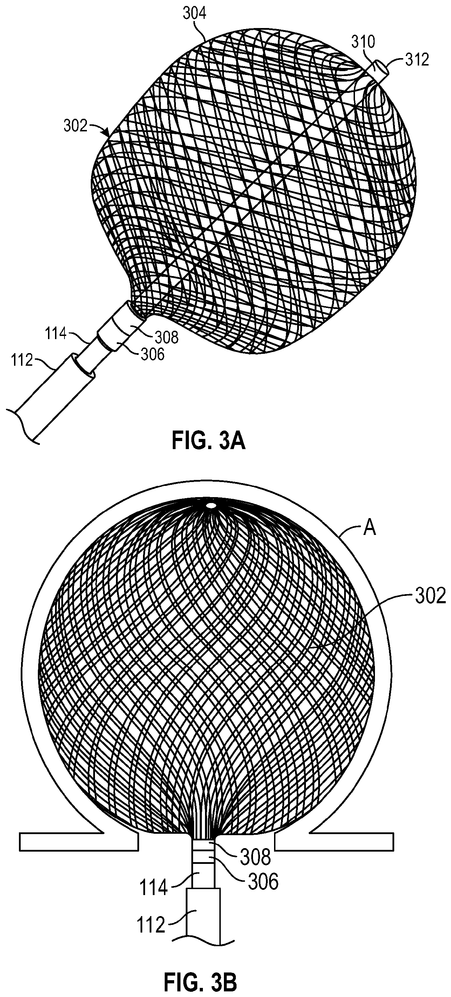

The neck cover 104 may comprise an expandable element having a low-profile or constrained state while positioned within a catheter (such as the second elongate shaft 112) for delivery to the aneurysm and an expanded, deployed state for positioning within the aneurysm. In some embodiments the neck cover 104 comprises a mesh 120 (shown schematically in FIG. 1B) and a connector 122 coupled to the mesh 120. The connector 122 is configured to be coupled to one or more components of the delivery system 102, such as the third elongate shaft 114 and/or extension 116. The mesh 120 can be formed of a resilient material and shape set such that upon exiting the second elongate shaft 112, the mesh 120 self-expands to a predetermined shape. The mesh 120 can have any shape or size in the expanded state that enables the mesh 120 to cover the aneurysm neck. In some embodiments, for example as shown in FIG. 2A, the mesh 120 can be configured to assume a bowl shape. Other shapes are possible, e.g., as described in connection with FIGS. 3A-3D below. The mesh 120 can have a porosity sufficient to prevent leakage of the embolic composition 152 into the parent vessel.

In some embodiments, the mesh 120 is formed of a plurality of braided filaments that have been heat-set to assume a predetermined shape when released from the constraints of the delivery catheter. The mesh 120 may be formed of metal wires, polymer wires, or both, and the wires may have shape memory and/or superelastic properties. The mesh 120 may be formed of 24, 32, 36, 48, 64, 72, 96, 128, or 144 filaments. The mesh 120 may be formed of a range of filament or wire sizes, such as wires having a diameter of from about 0.0004 inches to about 0.0020 inches, or of from about 0.0009 inches to about 0.0012 inches. In some embodiments, each of the wires or filaments have a diameter of about 0.0004 inches, about 0.0005 inches, about 0.0006 inches, about 0.0007 inches, about 0.0008 inches, about 0.0009 inches, about 0.001 inches, about 0.0011 inches, about 0.0012 inches, about 0.0013 inches, about 0.0014 inches, about 0.0015 inches, about 0.0016 inches, about 0.0017 inches, about 0.0018 inches, about 0.0019 inches, or about 0.0020 inches. In some embodiments, all of the filaments of the braided mesh 120 may have the same diameter. For example, in some embodiments, all of the filaments have a diameter of no more than 0.001 inches. In some embodiments, some of the filaments may have different cross-sectional diameters. For example, some of the filaments may have a slightly thicker diameter to impart additional strength to the braid. In some embodiments, some of the filaments can have a diameter of no more than 0.001 inches, and some of the filaments can have a diameter of greater than 0.001 inches. The thicker filaments may impart greater strength to the braid without significantly increasing the device delivery profile, with the thinner wires offering some strength while filling out the braid matrix density.

In some embodiments, the mesh 120 can be a non-braided structure, such as a laser-cut stent. Moreover, while the mesh 120 shown in FIGS. 2A-2E is a dual-layer mesh, in some embodiments the mesh 120 may comprise more or fewer layers (e.g., a single layer, three layers, four layers, etc.).

FIGS. 2A-2E illustrate an example method for treating an aneurysm using the system 100, in accordance with embodiments of the present technology. Referring first to FIG. 2A, a physician may begin by intravascularly advancing the second elongate shaft 112 towards an intracranial aneurysm A with the neck cover 104 in a low-profile, collapsed state and coupled to a distal end portion of the third elongate shaft 114. A distal portion of the second elongate shaft 112 may be advanced through a neck N of the aneurysm A to locate a distal opening of the second elongate shaft 112 within an interior cavity of the aneurysm A. The third elongate shaft 114 may be advanced distally relative to the second elongate shaft 112 to push the neck cover 104 through the opening at the distal end of the second elongate shaft 112, thereby releasing the neck cover 104 from the shaft 114 and enabling the neck cover 104 to self-expand into an expanded, deployed state.

FIG. 2A shows the neck cover 104 in an expanded, deployed state, positioned in an aneurysm cavity and still coupled to the third elongate shaft 114. In the expanded, deployed state, the neck cover 104 may generally conform to the curved inner surface of the aneurysm A. In some embodiments the neck cover 104 assumes a predetermined shape that is concave towards the aneurysm dome and encloses an interior region 124.

As illustrated in FIG. 2B, the embolic composition 152 can be injected through the third elongate shaft 114 and extension 116 to a space between the neck cover 104 and an inner surface of the aneurysm wall. In other embodiments, the embolic composition 152 can be delivered through another elongate shaft (not shown) separate from the third elongate shaft 114 and extension 116. As additional embolic composition 152 is delivered, it fills the interior region 124 and all or a portion of the volume of the aneurysm cavity. It may be beneficial to fill as much space in the aneurysm as possible, as leaving voids within the aneurysm sac may cause delayed healing and increased risk of aneurysm recanalization and/or rupture. While the scaffolding provided by the neck cover 104 across the neck helps thrombosis of blood form in any gaps and healing at the neck N, the substantial filling of the cavity can prevent rupture acutely and does not rely on the neck cover 104. In some embodiments, the embolic composition 152 may fill greater than 40%, 50%, 60%, 65%, 70%, 75%, 80%, 85%, or 90% of the aneurysm sac volume.

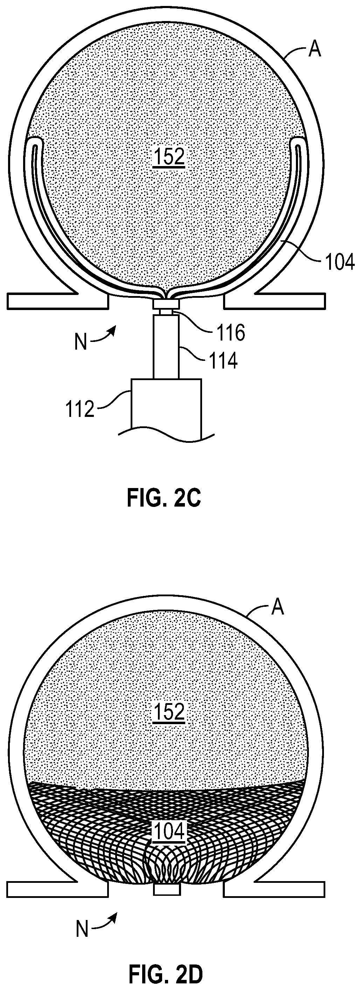

FIG. 2C is a cross-sectional view of the neck cover 104 still attached to the delivery system just after completion of delivery of the embolic composition 152. During and after delivery, the embolic composition 152 exerts a substantially uniform downward pressure (e.g., towards the parent vessel) on the neck cover 104 that further seals and stabilizes the neck cover 104 around the neck N of the aneurysm A. Moreover, the embolic composition 152 along the distal wall provides additional occlusion. In some embodiments, the embolic composition 152 completely or substantially completely occludes the pores of the adjacent layer or wall of the neck cover 104 such that blood cannot flow past the embolic composition 152 into the aneurysm cavity. It may be desirable to occlude as much of the aneurysm as possible, as leaving voids of gaps can enable blood to flow in and/or pool, which may continue to stretch out the walls of aneurysm A. Dilation of the aneurysm A can lead to recanalization and/or herniation of the neck cover 104 and/or embolic composition 152 into the parent vessel and/or may cause the aneurysm A to rupture. Both conditions can be fatal to the patient.

As shown in FIG. 2D, once delivery of the embolic composition 152 is complete, the delivery system 102 and/or third elongate shaft 114 can be detached from the neck cover 104 (electrolytically or mechanically) and withdrawn from the patient's body. In those embodiments comprising a separate elongate shaft for delivering the embolic composition 152, the elongate shaft can be withdrawn before, during, or after detachment of the third elongate shaft 114 from the neck cover 104.

Over time natural vascular remodeling mechanisms and/or bioabsorption of the embolic composition 152 (in embodiments where the embolic composition 152 is biodegradable) may lead to formation of a thrombus and/or conversion of entrapped thrombus to fibrous tissue within the internal volume of the aneurysm A. These mechanisms also may lead to cell death at a wall of the aneurysm and growth of new endothelial cells between and over the filaments of the neck cover 104. Eventually, the thrombus and the cells at the wall of the aneurysm may fully degrade, leaving behind a successfully remodeled region of the blood vessel.

In some embodiments, contrast agent can be delivered during advancement of the neck cover 104 and/or embolic composition 152 in the vasculature, deployment of the neck cover 104 and/or embolic composition 152 at the aneurysm A, and/or after deployment of the neck cover 104 and/or embolic composition 152 prior to initiation of withdrawal of the delivery system. The contrast agent can be delivered through the second elongate shaft 112, the conduit, or through another catheter or device commonly used to deliver contrast agent. The aneurysm (and devices therein) may be imaged before, during, and/or after injection of the contrast agent, and the images may be compared to confirm a degree of occlusion of the aneurysm. Alternatively, the contrast agent may be incorporated into the embolic composition 152, e.g., as described in Section II below.

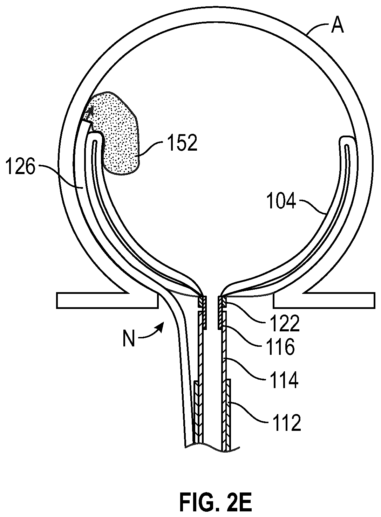

As shown in FIG. 2E, in some embodiments, the system 100 may comprise two separate elongate shafts (e.g., microcatheters), with one elongate shaft dedicated to delivery of the embolic composition 152 (e.g., a fourth elongate shaft 126), and the other elongate shaft dedicated to the delivery of the neck cover 104 (e.g., the third elongate shaft 114). In such embodiments, the fourth elongate shaft 126 can be fluidly coupled to the injector 154 to form at least part of the conduit for conveying the embolic composition 152 into the aneurysm A. The fourth elongate shaft 126 may be intravascularly advanced to the aneurysm A and through the neck N such that that a distal tip of the fourth elongate shaft 126 is positioned within the aneurysm cavity. In some embodiments, the fourth elongate shaft 126 may be positioned within the aneurysm cavity such that the distal tip of the fourth elongate shaft 126 is near the dome of the aneurysm A.

The third elongate shaft 114 containing the neck cover 104 may be intravascularly advanced to the aneurysm A and positioned within the aneurysm cavity adjacent the fourth elongate shaft 126. The neck cover 104 may then be deployed within the aneurysm sac. As the neck cover 104 is deployed, it pushes the fourth elongate shaft 126 outwardly towards the side of the aneurysm A, and when fully deployed the neck cover 104 holds or "jails" the fourth elongate shaft 126 between an outer surface of the neck cover 104 and the inner surface of the aneurysm wall.

The embolic composition 152 may then be delivered through the fourth elongate shaft 126 to a position between the inner surface of the aneurysm wall and the outer surface of the neck cover 104. For this reason, it may be beneficial to initially position the distal tip of the fourth elongate shaft 126 near the dome (or more distal surface) of the aneurysm wall. This way, the "jailed" fourth elongate shaft 126 will be secured by the neck cover 104 such that the embolic composition 152 gradually fills the open space in the aneurysm sac between the dome and the neck cover 104.

FIG. 3A illustrates a neck cover 302 configured in accordance with embodiments of the present technology, and FIGS. 3B-3D illustrate an example method for treating an aneurysm using the neck cover 302, in accordance with embodiments of the present technology. The neck cover 302 may be generally similar to the neck cover 104 of FIGS. 1A and 1B, and may incorporate any of the features of the neck cover 104 described herein. For example, the neck cover 302 may comprise an expandable element (e.g., a mesh 304) having a low-profile or constrained state while positioned within a catheter (such as the second elongate shaft 112) for delivery to the aneurysm and an expanded, deployed state for positioning within the aneurysm.

Referring first to FIG. 3A, the neck cover 302 can be deployed within an aneurysm, e.g., using the system 100 of FIGS. 1A and 1B. The proximal end of the neck cover 302 can be detachably coupled to a distal end of the third elongate shaft 114. For example, the third elongate shaft 114 can include a first connector 306, and the distal end of the neck cover 302 can include a second connector 308 configured to detachably couple with the first connector 306. The distal end of the neck cover 302 can be detachably coupled to a fourth elongate shaft 310 that is slidably disposed within the third elongate shaft 114. One or more connectors (not shown in FIG. 3A) can be included at the proximal end of the neck cover 302 and the fourth elongate shaft 310 to permit deformation and inversion of a portion of the neck cover 302, as discussed in more detail below.

The fourth elongate shaft 310 can be inserted into the system 100 before the neck cover 302 is expanded, while the neck cover 302 is expanded, or after the neck cover 302 has been expanded and then retracted to a partially inverted state, as discussed in more detail below. In some embodiments, the fourth elongate shaft 310 is configured to deliver an embolic composition 152 (e.g., received from the embolic kit 150 of FIG. 1A) though exit port 312 to a position beyond the proximal end of the partially inverted neck cover 302. As such, the embolic composition 152 can become positioned between the neck cover 302 and an inner wall of the aneurysm cavity, as described in greater detail below.

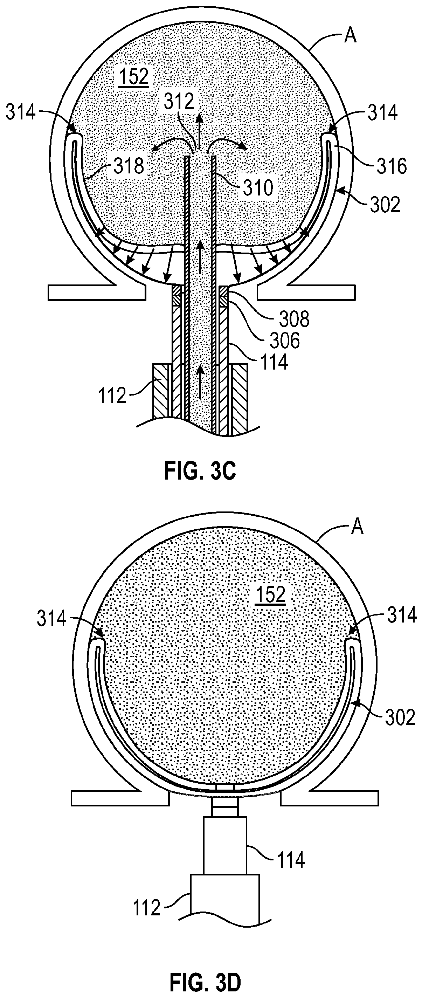

In FIG. 3B, the neck cover 302 has expanded to substantially fill the interior volume of aneurysm A. In FIG. 3C, the interior volume of aneurysm A has been largely filled with an embolic composition 152 injected through the exit port 312 at the end of the fourth elongate shaft 310. The embolic composition 152 can occupy the interior volume left by the retraction and partial inversion of the neck cover 302. As shown in FIG. 3C, an annular ridge 314 can separate an outer first portion 316 of the neck cover 302 from an inverted inner second portion 318, thereby forming a concave (e.g., bowl) shape. In the embodiment shown in FIG. 3C, pressure exerted by the embolic composition 152 can help invert the proximal end of the neck cover 302. However, the neck cover 302 can alternatively or additionally be manually retracted using a suitable wire or other member (not shown in FIG. 3C) prior to injection of the embolic composition 152. FIG. 3D shows the aneurysm A after it has been completely filled with the embolic composition 152. The embolic composition 152 can form a cohesive, solid hydrogel mass that seals the aneurysm A and facilitates healing thereof.

Although certain embodiments of the systems herein are described in connection with the treatment of aneurysms, such as cerebral aneurysms, this is not intended to be limiting, and the systems of the present technology can also be used in the treatment of other types of vascular defects, and/or in any other application involving delivery of an embolic composition into a space within a patient's body (e.g., middle meningeal artery embolization). In such embodiments, the system may be modified as appropriate for the particular use case, e.g., the neck cover may be replaced with a different type of occlusion device (e.g., a flow diverter) or the embolic composition can be used without any occlusion device (e.g., if physiological fluid flow at the treatment site is expected to be sufficiently low such that leakage of the embolic composition is not a significant concern).

### II. Embolic Compositions

The present technology provides compositions that form an injectable hydrogel suitable for partially or fully occluding an aneurysm or other space within the body. The composition can include a plurality of nanoparticles that are non-covalently crosslinked with each other and/or with a polymer to produce a hydrogel. The non-covalent crosslinking mechanism can confer shear-thinning properties to the hydrogel, e.g., the viscosity of the hydrogel decreases when shear stress is applied to the hydrogel and increases when the shear stress is removed, thus reducing the force needed to inject the hydrogel while allowing the hydrogel to form a solid cohesive mass once delivered into the aneurysm. The non-covalent crosslinking mechanism can also confer self-healing properties to the hydrogel, e.g., the non-covalent crosslinks can reform after disruption so that any cracking of the hydrogel that occurs can heal automatically.

### A. Nanoparticles

The hydrogels described herein can include a plurality of nanoparticles. The nanoparticles can be made out of any suitable biocompatible material (e.g., the material produces little or no toxicity, inflammatory response, or other undesirable side effects in vivo), which may or may not be biodegradable. In some embodiments, the nanoparticles are inorganic nanoparticles including one or more inorganic materials, such as silicates, oxides, halides, carbonates, phosphates, sulfide, sulfates, etc. For instance, the nanoparticles can be silicate nanoparticles that are composed of a silicate material, such as a layered silicate (also known as a phyllosilicate or nanoclay). Examples of layered silicates include smectites (e.g., laponite, montmorillonite, saponite, hectorite, bentonite), kaolinite, chlorite, and illite. The hydrogel can include a single type of nanoparticle or can include multiple different types of nanoparticles (e.g., two, three, four, five, or more different polymers).

In some embodiments, the nanoparticles have an average particle size within a range from 1 nm to 100 nm, 1 nm to 75 nm, 1 nm to 50 nm, 1 nm to 25 nm, 1 nm to 10 nm, 1 nm to 5 nm, 5 nm to 100 nm, 5 nm to 75 nm, 5 nm to 50 nm, 5 nm to 25 nm, 5 nm to 10 nm, 10 nm to 100 nm, 10 nm to 75 nm, 10 nm to 50 nm, 10 nm to 25 nm, 25 nm to 100 nm, 25 nm to 75 nm, 25 nm to 50 nm, 50 m to 100 nm, 50 nm to 75 nm, or 75 nm to 100 nm. The size of a particle can correspond to its diameter (for spherical or disk-shaped particles) or to its maximum linear dimension (for other particle shapes).

The nanoparticles can have any suitable form factor, such as disks, spheres, cubes, rods, tubes, fibers, etc. In some embodiments, the nanoparticles have an anisotropic shape. For example, the nanoparticles can be disk-shaped nanoparticles having a pair of planar faces and an edge connecting the faces. The diameter of the disk can be within a range from 10 nm to 50 nm, 15 nm to 35 nm, 20 nm to 30 nm, or 20 nm to 25 nm. The thickness of the disk can be within a range from 0.1 nm to 5 nm, 0.5 nm to 2 nm, 0.75 nm to 1 nm, or 1 nm to 1.25 nm.

In some embodiments, the nanoparticles form non-covalent crosslinks with each other. For example, the nanoparticles can be charged nanoparticles (e.g., at least at physiological pH (e.g., pH 7.4)) that are capable of interacting electrostatically with each other. The charged nanoparticles can include at least one charged portion, such as a cationic portion, an anionic portion, or both. The charge distribution of the nanoparticles can be anisotropic. For example, in embodiments where the nanoparticles are disk-shaped, the disk faces can be anionic while the disk edge is cationic, or vice-versa. The charged portion(s) of the nanoparticles can facilitate electrostatic interactions with other nanoparticles, e.g., a cationic portion of a nanoparticle can be attracted to an anionic portion of another nanoparticle, an anionic portion of a nanoparticle can be attracted to a cationic portion of another nanoparticle, a cationic portion of a nanoparticle can repel a cationic portion of another nanoparticle, and/or an anionic portion of a nanoparticle can repel an anionic portion of another nanoparticle. The charged portion(s) of the nanoparticles can alternatively or additionally facilitate electrostatic interactions with the polymeric component of the hydrogel, e.g., the cationic portion can be attracted to anionic functional groups on the polymer and/or the anionic portion can be attracted to cationic functional groups on the polymer. The electrostatic interactions between the nanoparticles and/or polymers can influence the overall properties of the hydrogel, as discussed further herein.

In some embodiments, the nanoparticles are laponite nanoparticles. Laponite has a disk-shaped structure with a diameter within a range from 20 nm to 30 nm, and a thickness of approximately 1 nm. Laponite has an anisotropic charge distribution, with the disk faces being anionic and the disk edge being cationic; due to the larger surfaces area of the faces, the overall charge is negative.

The concentration of the nanoparticles in the hydrogel can be varied as desired. Higher concentrations of nanoparticles can increase the stiffness and strength of the hydrogel, while lower concentrations of nanoparticles can increase the injectability of the hydrogel. In some embodiments, the concentration of the nanoparticles in the hydrogel as expressed in % w/w, individually or collectively, is greater than or equal to 1%, 2%, 5%, 6%, 8%, 10%, 12%, 14%, 15%, 16%, 18%, 20%, 22%, 25%, 30%, or 40%; and/or is no more than 50%, 40%, 30%, 25%, 22%, 20%, 18%, 16%, 15%, 14%, 12%, 10%, 8%, 6%, 5%, or 2%. The concentration of the nanoparticles in the hydrogel, individually or collectively, can be within a range from 1% to 30%, 5% to 25%, 10% to 20%, 10% to 15%, or 15% to 20%.

### B. Polymers

The hydrogels described herein can be composed of at least one polymer that forms non-covalent crosslinks with the nanoparticles. The non-covalent crosslinks between the polymer and nanoparticles can provide additional stability to support formation of a cohesive hydrogel. The polymer can include a functional group (e.g., a functional group of a repeating unit or an end group) that is capable of forming non-covalent interactions, such as electrostatic interactions (e.g., hydrogen bonds), hydrophobic associations, coordination complexes, or π-π stacking. For example, in embodiments where the nanoparticles include charged portions, the polymer can have at least one charged functional group (e.g., at least at physiological pH (e.g., pH 7.4)) that interacts electrostatically with the charged portions of the nanoparticles. In some embodiments, the polymer has an anionic functional group that interacts with a cationic portion of the nanoparticle, such as a carboxylic acid group, a phosphate group, a sulfate group, a sulfonate group, a nitrate group, etc. Acidic moieties such as carboxylic groups may additionally bond to endothelial cells at the treatment site to further improve occlusion stability. Alternatively or in combination, the polymer can have a cationic functional group that interacts with an anionic portion of the nanoparticle, such as an amine group. The hydrogel can include a single type of polymer or can include multiple different types of polymers (e.g., two, three, four, five, or more different polymers).

In some embodiments, the polymer is a synthetic polymer. Synthetic polymers can provide various advantages compared to naturally occurring polymers, such as improved control over molecular weight and molecular weight distribution, greater ease of preparation and chemical modification (e.g., including the ability to prepare copolymer to adjust solubility, rheological properties, etc.), reduced immunogenic potential, avoidance of animal-derived materials, and lower cost. The synthetic polymer can be biocompatible (e.g., the polymer produces little or no toxicity, inflammatory response, or other undesirable side effects in vivo), and may or may not be biodegradable. Examples of synthetic polymers that may be used in the hydrogels described include poly(acrylic acid), poly(methacrylic acid), poly(methyl methacrylate/methacrylic acid), oxidized poly(vinyl pyrrolidone), poly(ethylene glycol) diacid, carboxylic-acid functionalized poly(ethylene glycol) star polymer, poly(propylene glycol) diacid, poly(styrenesulfonic acid), poly(vinylsulfonic acid), poly(maleic acid), poly(butadiene/maleic acid), or poly(vinylphosphoric acid), or combinations (e.g., mixtures, copolymers) thereof. In some embodiments, the only polymers present in the hydrogel are synthetic polymers, and no naturally occurring polymers or derivatives of naturally occurring polymers (e.g., naturally occurring polymers that are synthetically functionalized) are used.

Optionally, the salt form of a polymer may be used, such as a sodium salt, an ammonium salt, etc. Examples of salt form polymers that may be used include poly(acrylic acid) sodium salt, poly(methacrylic acid) sodium salt, poly(methacrylic acid) ammonium salt, poly(styrenesulfonic acid) sodium salt, poly(vinylsulfonic acid) sodium salt, and poly(vinyl phosphoric acid) sodium salt. The salt form of a polymer can be produced, for example, by titrating the polymer with an appropriate base solution (e.g., a sodium salt of a polymer can be produced via titration with 0.01 N NaOH). In the salt form, at least some of the charged functional groups of the polymer can be neutralized by the salt counterion, thus allowing the overall charge of the polymer to be modified. For example, up to 1%, 2%, 5%, 10%, 15%, 20%, 25%, 30%, 40%, or 50% of the charged functional groups of the polymer can be neutralized. Adjustment of the degree of neutralization may affect the interactions between the polymer and the nanoparticles, e.g., a lower degree of neutralization may result in a less stiff and more injectable hydrogel, and a higher degree of neutralization can result in a stiffer and less injectable hydrogel.

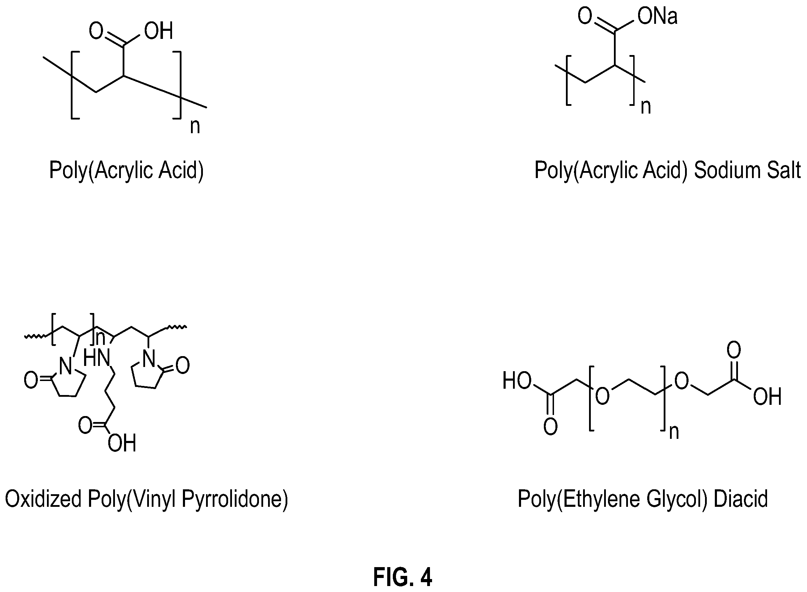

FIG. 4 illustrates the chemical structures of poly(acrylic acid), poly(acrylic acid) sodium salt, oxidized poly(vinyl pyrrolidone), and poly(ethylene glycol) diacid, which are representative examples of synthetic polymers that may be used in embodiments of the present technology.

In embodiments where the hydrogel includes poly(acrylic acid) or a salt thereof (e.g., poly(acrylic acid) sodium salt, the poly(acrylic acid) or salt thereof may be partially covalently crosslinked with itself, e.g., via heating to a temperature of at least 130 °C. Partial covalent crosslinking of the polymer may increase hydrogel stiffness and strength, for example. In other embodiments, however, the hydrogel may include non-covalent crosslinks only, without any covalent crosslinking between polymer chains.

The molecular weight of the polymer can be selected to provide desired hydrogel properties, e.g., higher molecular weight polymers have more interactions with the nanoparticles and thus produce stiffer and more cohesive hydrogels, while lower molecular weight polymers may have fewer interactions with the nanoparticles and thus produce softer and more injectable hydrogels. The molecular weight of the polymer can be sufficiently low to ensure that the polymer is soluble and can be mixed with the nanoparticles to produce a homogenous hydrogel. In some embodiments, the molecular weight of the polymer (weight average, number average, or viscosity average molecular weight) is greater than or equal to 100 kDa, 200 kDa, 300 kDa, 400 kDa, 450 kDa, 500 kDa, 550 kDa, 600 kDa, 650 kDa, 700 kDa, 750 kDa, 800 kDa, 850 kDa, 900 kDa, 950 kDa, 1 MDa, 1.25 MDa, 1.5 MDa, 1.75 MDa, 2 MDa, 2.25 MDa, 2.5 MDa, or 2.75 MDa; and/or is less than or equal to 3 MDa, 2.75 MDa, 2.5 MDa, 2.25 MDa, 2 MDa, 1.75 MDa, 1.5 MDa, 1.25 MDa, 1 MDa, 950 kDa, 900 kDa, 850 kDa, 800 kDa, 750 kDa, 700 kDa, 650 kDa, 600 kDa, 550 kDa, 500 kDa, 450 kDa, 400 kDa, 300 kDa, or 200 kDa. The molecular weight of the polymer can be within a range from 100 kDa to 2 MDa, 100 kDa to 500 kDa, 250 kDa to 650 kDa, 500 kDa to 1 MDa, 1 MDa to 1.5 MDa, or 1.5 MDa to 2 MDa.

The dispersity of the polymer (which can be calculated from the ratio of the weight average molecular weight to the number average molecular weight) can be less than or equal to 3, 2.5, 2.2, 2, 1.8, 1.6, 1.5, 1.4, 1.3, 1.2, 1. 1, or 1.05.

The concentration of the polymer in the hydrogel can be varied as desired. Higher concentrations of polymer may increase the interactions with the nanoparticles and thus produce stiffer, stronger, and more viscous hydrogels, while lower concentrations of polymer may result in fewer interactions with the nanoparticles and thus produce softer and more injectable hydrogels. In some embodiments, the concentration of the polymer(s) in the hydrogel (% w/w), individually or collectively, is greater than or equal to 0.1%, 0.2%, 0.3%, 0.4%, 0.5%, 0.6%, 0.7%, 0.8%, 0.9%, 1%, 1.5%, 2%, 2.5%, 3%, 3.5%, 4%, or 4.5%; and/or is no more than 5%, 4.5%, 4%, 3.5%, 3%, 2.5%, 2%, 1.5%, 1%, 0.9%, 0.8%, 0.7%, 0.6%, 0.5%, 0.4%, 0.3%, or 0.2%. The concentration of the polymer(s) in the hydrogel, individually or collectively, can be within a range from 0.1% to 5%, 0.1% to 2%, 0.1% to 1%, 0.1% to 0.5%, 0.5% to 5%, 0.5% to 2%, 0.5% to 1%, 1% to 5%, 1% to 2%, or 2% to 5%.

### C. Contrast Agents

In some embodiments, the hydrogels described herein include at least one contrast agent that allows for visualization of the hydrogel during and/or after injection into the treatment site. For example, the contrast agent can be configured for radiographic imaging. Examples of contrast agents that may be used include tantalum, bismuth trioxide, bismuth oxychloride, tungsten, tungsten carbide, barium sulfate, gadolinium, iodized oil (e.g., LIPIODOL^{®}), iohexol (e.g., OMNIPAQUE^{™} from GE Healthcare), iopamidol (e.g., ISOVUE^{™} from Bracco Diagnostics, Inc.), ioxilan, iopromide, iodixanol (e.g., VISIPAQUE^{™} from GE Healthcare), iobitridol, ioversol, diatrizoate (e.g., HYPAQUE^{™} from GE Healthcare), metrizoate, iothalamate (e.g., CONRAY^{™} from Covidien), ioxaglate, iothalamate/meglumine, ioxaglate/meglumine, diatrizoate/meglumine, iodomide sodium, metrizamide, or combinations thereof. The hydrogel can include a single type of contrast agent or can include a plurality of different types of contrast agents (e.g., two, three, four, five, or more different types of contrast agents).

The concentration of the contrast agent(s) in the hydrogel (% w/w), individually or collectively, can be greater than or equal to 10%, 20%, 30%, 40%, 50%, 60%, 70%, 80%, or 90%, and/or no more than 90%, 80%, 70%, 60%, 50%, 40%, 30%, 20%, or 10%. In some embodiments, the concentration of the contrast agent(s) in the hydrogel, individually or collectively, is within a range from 10% to 90%, 10% to 80%, 10% to 60%, 10% to 50%, 10% to 40%, 10% to 30%, 20% to 90% 20% to 80%, 20% to 60%, 20% to 50%, 20% to 40%, 20% to 30%, 25% to 75%, 25% to 50%, 30% to 80%, 30% to 50%, 50% to 75% or 75% to 95%.

Optionally, after the hydrogel is implanted into the treatment site, the contrast agent may gradually diffuse out of the hydrogel, thus resulting in diminishing radiopacity over time. This approach may be advantageous where the contrast agent produces imaging artifacts that may interfere with subsequent imaging of the treatment site. In some embodiments, the hydrogel is configured to release at least 10%, 20%, 30%, 40%, 50%, 60%, 70%, 80%, 90%, 95%, or 99% of the initial loading contrast agent within a target time period after implantation, where the target time period is at least 1 day, 2 days, 3 days, 4 days, 5 days, 6 days, 7 days, 2 weeks, 3 weeks, 4 weeks, 1 month, 2 months, or 3 months after implantation. In other embodiments, however, the contrast agent may remain within the hydrogel without leaching out after implantation.

In other embodiments, the contrast agent is optional and may be omitted from the hydrogel. In such embodiments, the degree of occlusion of the treatment site by the hydrogel may be assessed using other techniques, such as based on extent of inversion of neck cover by the hydrogel (e.g., as discussed above with respect to FIGS. 3A-3D).

### D. Solvents

The hydrogels disclosed herein can also include at least one solvent. The solvent can be a biocompatible aqueous solution, such as water (e.g., distilled water, deionized water), a saline solution (e.g., normal saline, Ringer's lactate solution, phosphate-buffered saline), etc. The solvent can optionally be supplied as a part of other components of the hydrogel, such as the contrast agent.

The concentration of the solvent in the hydrogel (% w/w) can be greater than or equal to 10%, 20%, 30%, 40%, 50%, 60%, 70%, 80%, or 90%, and/or no more than 90%, 80%, 70%, 60%, 50%, 40%, 30%, 20%, or 10%. In some embodiments, the concentration of the solvent in the hydrogel is within a range from 10% to 90%, 10% to 80%, 10% to 60%, 10% to 50%, 10% to 40%, 10% to 30%, 20% to 90% 20% to 80%, 20% to 60%, 20% to 50%, 20% to 40%, 20% to 30%, 25% to 75%, 25% to 50%, 30% to 80%, 30% to 50%, 50% to 75%, or 75% to 95%.

### E. Hydrogel Properties

The hydrogels described herein can be configured for intravascular delivery into a treatment site (e.g., an aneurysm or other vascular defect) via injection through an elongate shaft (e.g., a microcatheter or other delivery catheter). In some embodiments, the hydrogels disclosed herein exhibit shear-thinning properties suitable for injection into the treatment site via an elongate shaft having a relatively small inner diameter, such as a microcatheter having an inner diameter less than or equal to 0.02 inches, 0.015 inches, 0.014 inches, 0.013 inches, 0.012 inches, 0.011 inches, or 0.01 inches. The viscosity of the hydrogel can decrease when subjected to shear stress (e.g., during injection through the delivery catheter) to allow the hydrogel to be delivered into the treatment site with relatively low injection forces. When the shear stress is removed (e.g., after the hydrogel has exited the delivery catheter), the viscosity of the hydrogel can increase to allow the hydrogel to form a unitary, cohesive, solid or semi-solid mass to fill and seal the treatment site without dispersing or dissolving when exposed to in vivo conditions (at least until biodegradation and/or bioresorption of the polymer occurs, if applicable). As discussed above, this approach can reduce the likelihood of the hydrogel leaking out of the treatment site during and/or after delivery, thus lowering the risk of patient complications such as stroke.

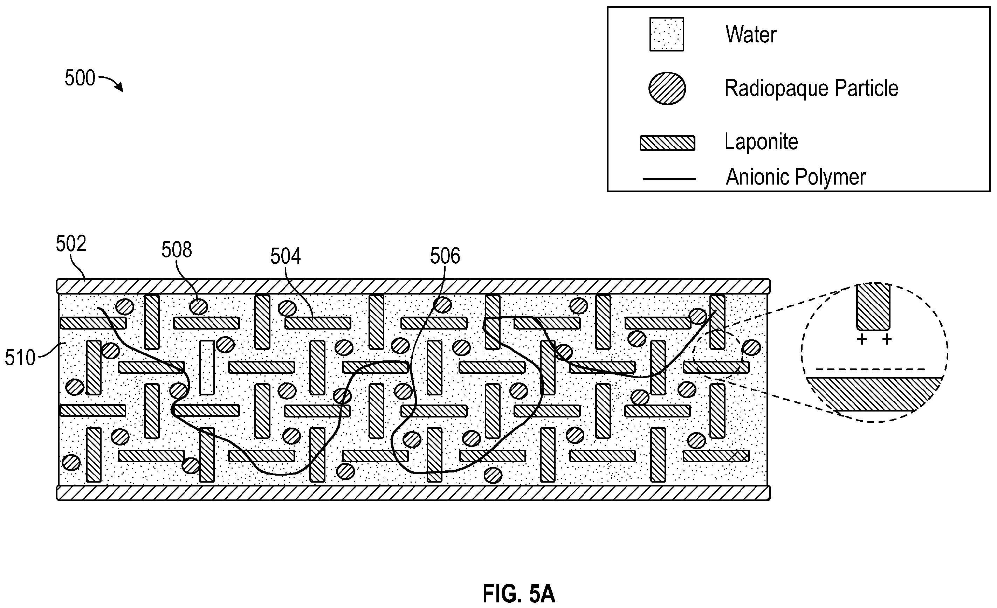

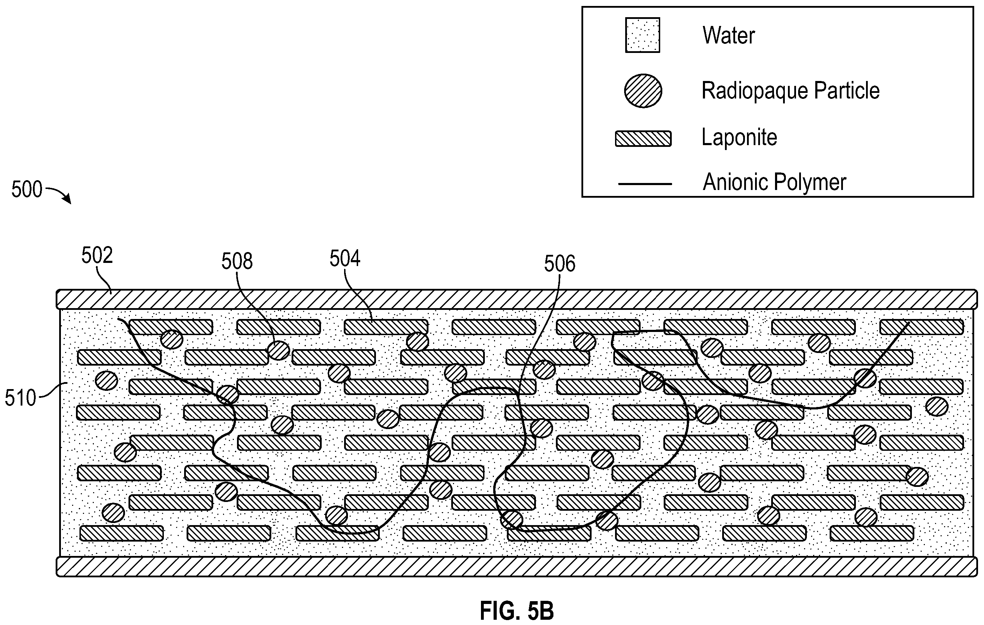

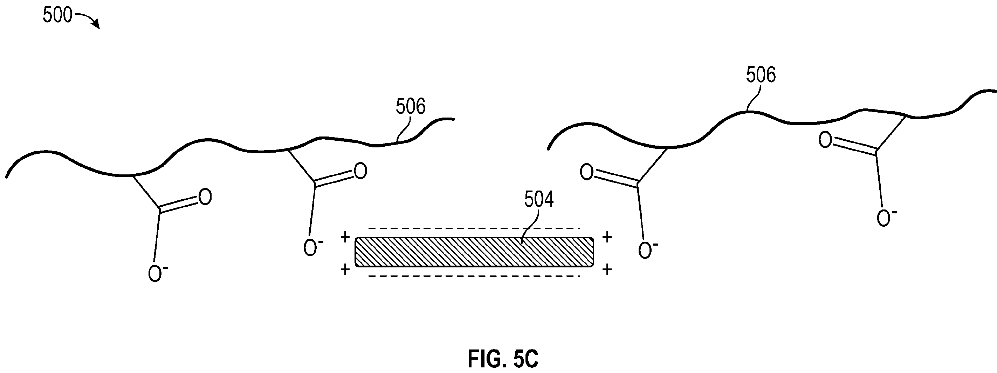

FIGS. 5A-5C are schematic illustrations of a hydrogel 500 having shear-thinning properties, in accordance with embodiments of the present technology. Specifically, FIG. 5A is a side cross-sectional view of the hydrogel 500 in an injector 502 while no injection force is applied, FIG. 5B is a side cross-sectional view of the hydrogel 500 in the injector 502 while an injection force is applied, and FIG. 5C illustrates physical crosslinking between hydrogel components.

Referring first to FIG. 5A, the hydrogel 500 can be composed of a plurality of laponite nanoparticles 504, an anionic polymer 506 (e.g., a polymer having carboxylic acid functional groups), a contrast agent 508 (e.g., radiopaque particles such as tantalum), and a solvent 510 (e.g., water). When no force is being applied to the hydrogel 500, the laponite nanoparticles 504 can form a "house-of-cards" 3D structure due to electrostatic interactions between the cationic disk edges and the anionic disk faces (shown in inset view). Moreover, as depicted in FIG. 5C, the anionic functional groups of the anionic polymer 506 can interact electrostatically with the cationic disk edges of the laponite nanoparticles 504, thus further stabilizing the nanoparticle network. Accordingly, the hydrogel 500 can exhibit solid-like behavior (e.g., the storage modulus of the hydrogel 500 is greater than the loss modulus of the hydrogel 500) with a relatively high viscosity.

Referring next to FIG. 5B, when force is applied to the hydrogel 500 during injection, the force can disrupt the electrostatic interactions between the laponite nanoparticles 504 and cause the laponite nanoparticles 504 to become aligned parallel with each other and to the direction of flow (this configuration can produce less resistance to flow and lower energy due to the anisotropic nanoparticle shape). Accordingly, the hydrogel 500 can exhibit liquid-like behavior (e.g., the loss modulus of the hydrogel 500 is greater than the storage modulus of the hydrogel 500) with a relatively low viscosity. The anionic polymer 506 may still interact electrostatically with the laponite nanoparticles 504, although some of the polymer-nanoparticle interactions may break and reform due to the applied force.

Once the applied force is removed (e.g., after the hydrogel 500 has been delivered into the treatment site), the laponite nanoparticles 504 can transition back into the house-of-cards 3D structure shown in FIG. 5A, thus causing the hydrogel 500 to return to a solid-like state.

In some embodiments, the hydrogels described herein can be used without carrying out any preliminary mixing and/or crosslinking of precursor materials. In some embodiments, the hydrogels herein can be delivered into the patient's body without any prior mixing and/or crosslinking steps that occur within 1 minute, 2 minutes, 5 minutes, 10 minutes, 30 minutes, 1 hour, 2 hours, 4 hours, 8 hours, 12 hours, 24 hours, 36 hours, 48 hours, 3 days, 4 days, 5 days, 6 days, 1 week, 2 weeks, 3 weeks, 4 weeks, 2 months, 3 months, 6 months, or 1 year before delivery. Instead, the hydrogels can be provided in an injectable, preformed state in a sterilized package (e.g., a vial or preloaded syringe) that is immediately ready for use. Accordingly, in contrast to conventional approaches, the hydrogel can be used without relying on an in situ polymerization reaction, crosslinking reaction, or other chemical reaction that may occur while the hydrogel is being injected, which may cause the hydrogel to become too viscous to be injected through a microcatheter. Excessive viscosity may cause plugging or rupture of the microcatheter, and may delay or even prevent a surgical aneurysm repair procedure. Moreover, the hydrogels herein can transition between liquid-like and solid-like states based solely on whether force is being applied to the hydrogel, rather than requiring a separate trigger (e.g., temperature, pH, salt concentration) to trigger the liquid-solid transition, which can improve ease of use, reliability, and consistency.

The properties of the hydrogel can be controlled based at least in part on the ratio of the nanoparticles to the polymer in the hydrogel. In some embodiments, the ratio of the nanoparticles to the polymer in the hydrogel by weight is at least 1, 2, 3, 4, 5, 10, 15, 20, 25, 30, 35, 40, 45, or 50; and or is no more than 60, 50, 45, 40, 35, 30, 25, 20, 15, 10, 5, 4, 3, 2, or 1. The ratio of the nanoparticles to the polymer in the hydrogel by weight can be within a range from 1 to 10, 1 to 5, 5 to 10, 5 to 50, 10 to 20, or 20 to 50. Without wishing to be bound by theory, it is hypothesized that in embodiments where the polymer and nanoparticles are oppositely charged, the hydrogel strength and cohesion may be enhanced if the nanoparticle: polymer ratio is selected so that the charge on the polymer is balanced with the charge on the nanoparticles (e.g., the amount of anionic charge on the polymer is equal or similar to the amount of cationic charge on the nanoparticles). If the nanoparticle: polymer ratio is too high, there may be insufficient non-covalent crosslinking between the polymer and nanoparticles, resulting in a weaker hydrogel. If the nanoparticle:polymer ratio is too low, the excess polymer may increase the overall viscosity of the hydrogel and dominate over the shear-thinning properties of the nanoparticles, thus reducing injectability of the hydrogel. The charge balance associated with a particular nanoparticle:polymer ratio can be determined and controlled, for example, based on pH measurements of the hydrogel (e.g., laponite nanoparticles in water have a pH of approximately 10, and PAA is slightly acidic).

In some embodiments, the hydrogels herein have a first, higher viscosity at a first, lower shear rate, and a second, lower viscosity at a second, higher shear rate. For example, at a temperature of 37 °C, the hydrogel can have a viscosity greater than or equal to 10 Pa-s, 50 Pa-s, 100 Pa-s, 250 Pa-s, 500 Pa-s, 750 Pa-s, 1000 Pa-s, 2000 Pa-s, or 5000 Pa-s when the shear rate is less than or equal to 10⁰ s⁻¹, 10⁻¹ s⁻¹, 10⁻² s⁻¹, or 10⁻³ s⁻¹; and can have a viscosity less than or equal to 10 Pa-s, 5 Pa-s, 4 Pa-s, 3 Pa-s, 2 Pa-s, 1 Pa-s, 0.5 Pa-s, 0.1 Pa-s, 0.05 Pa-s, 0.02 Pa-s, or 0.01 Pa-s when the shear rate is greater than or equal to 10⁰ s⁻¹, 10¹ s⁻¹, 10² s⁻¹, or 10³ s⁻¹. The viscosity may change by at least 1, 2, 3, or 4 orders of magnitude across a range of shear rates from 10⁻³ s⁻¹ to 10³ s⁻¹. The viscosity of the hydrogel under shear can be measured via any suitable technique, such as using an oscillatory parallel plate rheometer (e.g., 40 mm plate diameter) operating in shear mode at a suitable temperature (e.g., 37 °C).

In some embodiments, the hydrogels herein have a storage modulus within the linear viscoelastic region that is within a range from 1 MPa to 10 MPa, 1 MPa to 5 MPa, 1 MPa to 2 MPa, 2 MPa to 10 MPa, 2 MPa to 5 MPa, or 5 MPa to 10 MPa. In some embodiments, the hydrogels herein have a loss modulus within the linear viscoelastic region that is within a range from 100 Pa to 1000 Pa, 100 Pa to 500 Pa, 100 Pa to 200 Pa, 200 Pa to 1000 Pa, 200 Pa to 500 Pa, or 500 Pa to 1000 Pa. The storage modulus can be greater than the loss modulus of the hydrogel over a wide range of angular frequencies (e.g., from 0.1 rad/sec to 400 rad/sec), thus indicating that the hydrogel is stable, does not exhibit sol-gel transitions, and remains primarily elastic (solid-like), which may be desirable for a cohesive hydrogel. For example, the ratio of the loss modulus to the storage modulus (tan(delta) of the hydrogel can be within a range from 0.1 to 0.9, 0.1 to 0.5, or 0.3 to 0.4. The storage and loss moduli of the hydrogel can be measured via any suitable technique, such as using an oscillatory parallel plate rheometer (e.g., 40 mm plate diameter) operating in shear mode at a suitable temperature (e.g., 37 °C). The storage and loss moduli can be measured within the linear viscoelastic region of the hydrogel, where the linear viscoelastic region of the hydrogel corresponds to a critical strain value that is less than or equal to 5%, 4%, 3%, 2%, or 1%. The storage and loss moduli can be measured at a suitable angular frequency, such as an angular frequency of 0.1 rad/s, 1 rad/s, 10 rad/s, or 100 rad/s.

In some embodiments, the maximum force to inject the hydrogel is less than or equal to 20 N, 15 N, 10 N, 5 N, or 1 N. The injection force can be measured using any suitable technique, such as based on the amount of force to inject the hydrogel through a 25-gauge needle at an injection speed of 0.3 mL/min at 22 °C.

### F. Methods

In some embodiments, the present technology provides a method for preparing a hydrogel for occluding a treatment site, such as an aneurysm or other vascular defect. The method can include combining a plurality of nanoparticles (e.g., silicate nanoparticles) with a polymer (e.g., a synthetic polymer) in a solvent (e.g., water) to form a hydrogel including (1) non-covalent crosslinks (e.g., electrostatic interactions) between the nanoparticles and (2) non-covalent crosslinks (e.g., electrostatic interactions) between the nanoparticles in the polymer. Optionally, the composition can include additional components, such as a contrast agent, which may be added concurrently with the nanoparticles and polymer, or after the nanoparticles have already been combined with the polymer to form the hydrogel. In some embodiments, the composition includes a plurality of nanoparticles, a polymer, a contrast agent, and a solvent. In some embodiments, the composition includes a plurality of nanoparticles, a polymer, and a solvent, without any contrast agent. The types and concentrations of nanoparticles, polymer, contrast agent, and solvent can be varied as desired, e.g., as discussed in Sections II.A-II.D above.

In some embodiments, the present technology provides a method for treating a patient using a hydrogel as described herein. The method can include delivering a hydrogel to a treatment site. For example, the hydrogel can be delivered into an aneurysm to partially or fully occlude the aneurysm. The hydrogel can be delivered via injection, e.g., via a catheter or other elongate shaft that is introduced to the treatment site via the vasculature. As discussed herein, the hydrogel can have shear-thinning properties so that the hydrogel flows like a liquid during injection and forms a solid or semi-solid mass after delivery into the treatment site without leaking.

Optionally, the hydrogel can be used in combination with another device, such as a neck cover for an aneurysm (e.g., as described in Section I above). In such embodiments, the neck cover can be positioned within the aneurysm before the hydrogel is delivered into the aneurysm. The hydrogel can have sufficient stiffness and cohesive strength such that when the hydrogel is delivered into the aneurysm, the hydrogel pushes downward against the neck cover to ensure that the interior cavity of the aneurysm is substantially completely filled, without leaking through the neck cover.

In some embodiments, the methods herein can be used to treat other types of diseases or conditions besides aneurysms. Examples of diseases and conditions that are also applicable to the present technology include arteriovenous malformations (e.g., brain arteriovenous malformations), arteriovenous fistulas, tumors (e.g., via occlusion of vessel(s) feeding a tumor), chronic subdural hematomas (e.g., via occlusion of the middle meningeal artery), perivascular leaks, varicose veins (e.g., via occlusion of one or more truncal veins such as the great saphenous vein), hemorrhoids, and sealing endoleaks adjacent to artificial heart valves, covered stents, and abdominal aortic aneurysm devices.

### III. Examples

The following examples are included to further describe some aspects of the present technology, and should not be used to limit the scope of the technology.

### Example 1: Preparation and Characterization of Composite Hydrogels

This example describes the preparation and characterization of hydrogels composed of laponite nanoparticles and poly(acrylic acid) (PAA).

Hydrogels were formulated using laponite nanoparticles, PAA (viscosity average molecular weights of 2 kDa, 450 kDa, 1.25 MDa, or 3 MDa), and water. To formulate the hydrogels, a laponite/water suspension and a corresponding PAA solution were prepared. Different ratios of the laponite/water suspension and PAA solution were mixed with the addition of water to prepare the formulations. The suspensions were sonicated for several minutes to ensure a homogeneous hydrogel. The various formulations are shown in Tables 1-4 below. Injection force measurements (Formulations 8, 10, 12, and 17 in Table 1) were conducted by injecting the hydrogel from a syringe through a 25-gauge needle at a rate of 0.3 mL/min using an Instron testing system with a custom-made fixture to push the syringe plunger.

**Table 1: Evaluation of Hydrogels with 2 kDa PAA**

| **Formulation** | **Water (% w/w)** | **Laponite (% w/w)** | **PAA (% w/w)** | **Laponite/PAA Ratio** | **Results** |
|---|---|---|---|---|---|
| 1 | 60 | 30 | 10 | 3.0 | No gelation |
| 2 | 69 | 30 | 1 | 30.0 | Weak gel |
| 3 | 70.6 | 22 | 7.4 | 3.0 | No gelation |
| 4 | 74.8 | 25 | 0.2 | 125.0 | No gelation |
| 5 | 75.1 | 22 | 2.9 | 7.6 | Weak gel |
| 6 | 77 | 16 | 7 | 2.3 | No gelation |
| 7 | 80 | 1 | 19 | 0.1 | No gelation |
| 8 | 80 | 19 | 1 | 19.0 | Gel, 2.96 N injection force |
| 9 | 80 | 10 | 10 | 1.0 | No gelation |
| 10 | 83 | 13 | 4 | 3.3 | Gel, 22.4 N injection force |
| 11 | 83 | 4 | 13 | 0.3 | Very weak gel |
| 12 | 86 | 7 | 7 | 1.0 | Weak gel, 9.8 N injection force |
| 13 | 86 | 13.8 | 0.2 | 69.0 | Weak gel |
| 14 | 87 | 3 | 10 | 0.3 | No gelation |
| 15 | 88.1 | 9 | 2.9 | 3.1 | Weak gel |
| 16 | 89.5 | 0.5 | 10 | 0.1 | No gelation |
| 17 | 92 | 4 | 4 | 1.0 | Weak gel, 16.9 injection force |
| 18 | 95 | 4 | 1 | 4.0 | Weak gel |
| 19 | 98 | 1 | 1 | 1.0 | No gelation |

**Table 2: Evaluation of Hydrogels with 450 kDa PAA**

| **Formulation** | **Water (% w/w)** | **Laponite (% w/w)** | **PAA (% w/w)** | **Laponite/PAA Ratio** | **Results** |
|---|---|---|---|---|---|
| 1 | 75 | 20 | 5 | 4.0 | Good, cohesive gel |
| 2 | 79.8 | 20 | 0.2 | 100.0 | Weak gel |
| 3 | 83.35 | 15.25 | 1.4 | 10.9 | Good gel |
| 4 | 86.9 | 10.5 | 2.6 | 4.0 | No gelation |
| 5 | 90.45 | 5.75 | 3.8 | 1.5 | No gelation |
| 6 | 90.95 | 15.25 | 3.8 | 4.0 | Good gel |
| 7 | 92.85 | 5.75 | 1.4 | 4.1 | Gel |
| 8 | 94 | 1 | 5 | 0.2 | No gelation |
| 9 | 98.8 | 1 | 0.2 | 5.0 | Weak gel |

**Table 3: Evaluation of Hydrogels with 1.25 MDa PAA**

| **Formulation** | **Water (% w/w)** | **Laponite (% w/w)** | **PAA (% w/w)** | **Laponite/PAA Ratio** | **Results** |
|---|---|---|---|---|---|
| 1 | 79.79 | 20 | 0.21 | 95.2 | Weak gel |
| 2 | 79.98 | 20 | 0.02 | 1000.0 | No gelation |
| 3 | 84.2 | 15.5 | 0.31 | 50.0 | Good gel |
| 4 | 84.39 | 15.5 | 0.12 | 129.2 | No gelation |
| 5 | 88.79 | 11 | 0.21 | 52.4 | Weak gel |
| 6 | 93.2 | 6.5 | 0.31 | 21.0 | Cohesive gel |
| 7 | 93.39 | 6.5 | 0.12 | 54.2 | Weak gel |
| 8 | 97.6 | 2 | 0.4 | 5.0 | Cohesive gel |
| 9 | 97.98 | 2 | 0.02 | 100.0 | No gelation |

**Table 4: Evaluation of Hydrogels with 3 MDa PAA**

| **Formulation** | **Water (% w/w)** | **Laponite (% w/w)** | **PAA (% w/w)** | **Laponite/PAA Ratio** | **Results** |
|---|---|---|---|---|---|
| 1 | 83.2 | 15.25 | 1.55 | 9.8 | Gel |
| 2 | 78 | 20 | 2 | 10.0 | Non-homogenous gel |
| 3 | 79.8 | 20 | 0.2 | 100.0 | No gelation |
| 4 | 84.1 | 15.25 | 0.65 | 23.5 | No gelation |
| 5 | 88.4 | 10.5 | 1.1 | 9.5 | No gelation |
| 6 | 92.7 | 5.75 | 1.55 | 3.7 | Weak gel |
| 7 | 93.6 | 5.75 | 0.65 | 8.8 | No gelation |
| 8 | 97 | 1 | 2 | 0.5 | Weak gel |
| 9 | 98.8 | 1 | 0.2 | 5.0 | No gelation |

All of the hydrogels formulated with 2 kDa PAA (Table 1) did not exhibit sufficient cohesive strength. Some of the formulations with 450 kDa PAA (Table 2) and 1.25 MDa PAA (Table 3) did produce good gels, although cohesive strength could still be improved. Due to the poor solubility of 3 MDa PAA, the formulations with this polymer included low polymer amounts and produced cohesively weak gels.

Formulation 3 of Table 3, which included 15.5% laponite, 0.31% 1.25 MDa PAA, and 84.2% water, exhibited a good balance of properties and produced successful occlusion in a simulated silicone aneurysm model.

Overall, these results demonstrate that the laponite/PAA ratio, the molecular weight of the PAA MW, and the concentration of each component affected the gel formation and mechanical properties. Without wishing to be bound by theory, it is hypothesized that low molecular weight PAA (e.g., 2 kDa) did not provide sufficient non-covalent crosslinking between the polymer and nanoparticles and thus resulted in weak hydrogels. Above 450 kDa, the mechanical properties of the hydrogel improved as the longer polymer chains interacted with higher number of nanoparticles, with the 1 MDa PAA producing good gel cohesion. Hydrogel formulated with 3 MDa PAA exhibited challenges with polymer dissolution as well as high viscosity leading to inhomogeneous and harder to inject hydrogels after mixing with laponite. For a given PAA polymer, it is hypothesized that the matching of the opposite charges of PAA and laponite resulted in stronger hydrogels; an excess of laponite appeared to produce a loosely crosslinked hydrogel, while an excess of polymer appeared to produce free flowing polymer chains and poor mechanical properties.

### Example 2: Rheological Properties of Composite Hydrogels

This example describes the rheological characterization of hydrogels composed of laponite nanoparticles and poly(acrylic acid) (PAA).

Hydrogels were formulated using 12.4% w/w laponite nanoparticles, 0.3% w/w PAA (viscosity average molecular weight of 1.25 MDa), 67.3% w/w water, and 20 % w/w tantalum. The hydrogels were prepared according to the protocol in Example 1 above.

Rheological testing was performed using an oscillatory parallel plate rheometer with 40 mm diameter stainless steel plates. Testing was conducted at 37 °C.

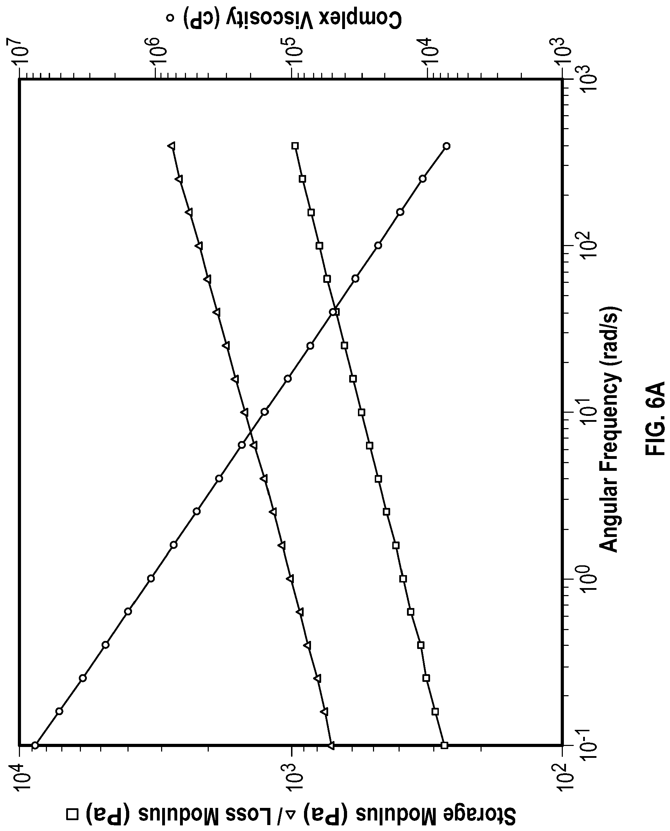

FIG. 6A is a graph of storage modulus, loss modulus, and complex viscosity of the hydrogel at 3% strain. The formulation was stable (no sol-gel transitions) across a wide range of frequencies (0.1-400 rad/sec). The storage modulus remained higher than the loss modulus across this range of frequencies, thus showing that the formulation had primarily elastic behavior, did not flow under these conditions, and remained solid-like.

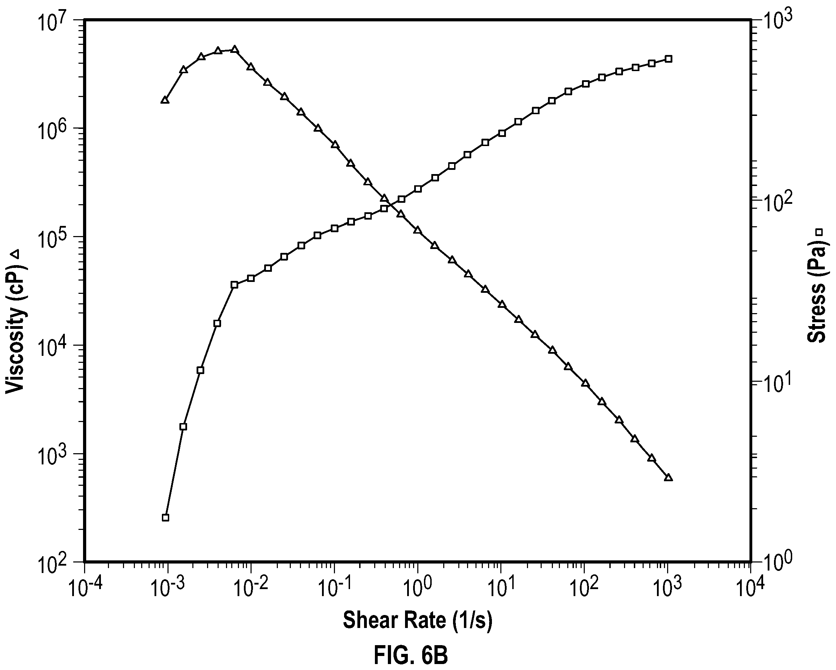

FIG. 6B is a graph illustrating shear-thinning behavior of the hydrogel at 37 °C. At low shear rates (less than 10⁻² sec⁻¹), the viscosity was above 1,000,000 cP (1000 Pa-s). At high shear rates (greater than 10² sec⁻¹), the viscosity decreased to below 600 cP (0.6 Pa-s).

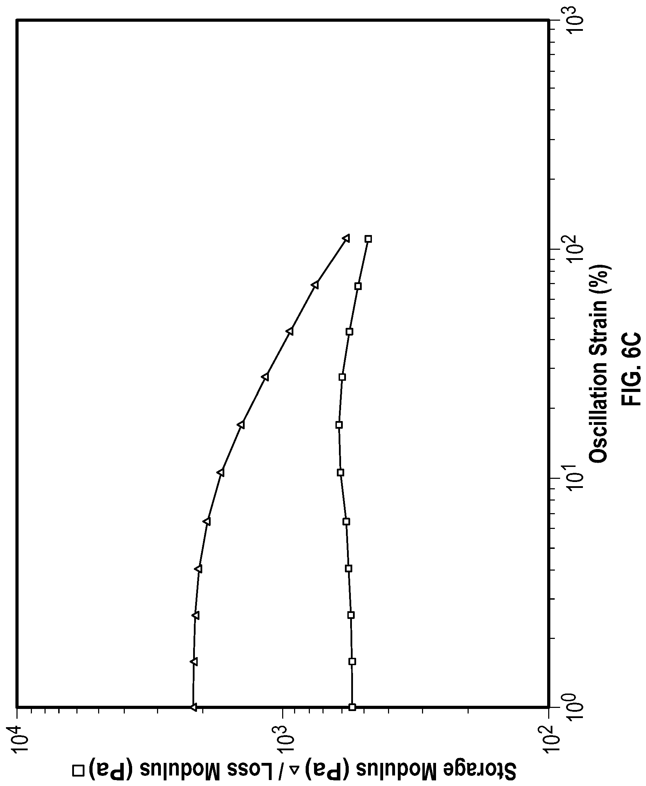

FIG. 6C is a graph illustrating the linear viscoelastic properties of the hydrogel at a frequency of 10 rad/sec. The hydrogel showed linear viscoelastic properties below 3% strain, where the storage modulus was 2,100 Pa ± 200 Pa, and the loss modulus was 600 Pa ± 100 Pa. These values correspond to the desired hydrogel properties for occlusion (e.g., sufficient cohesive strength and injectability).

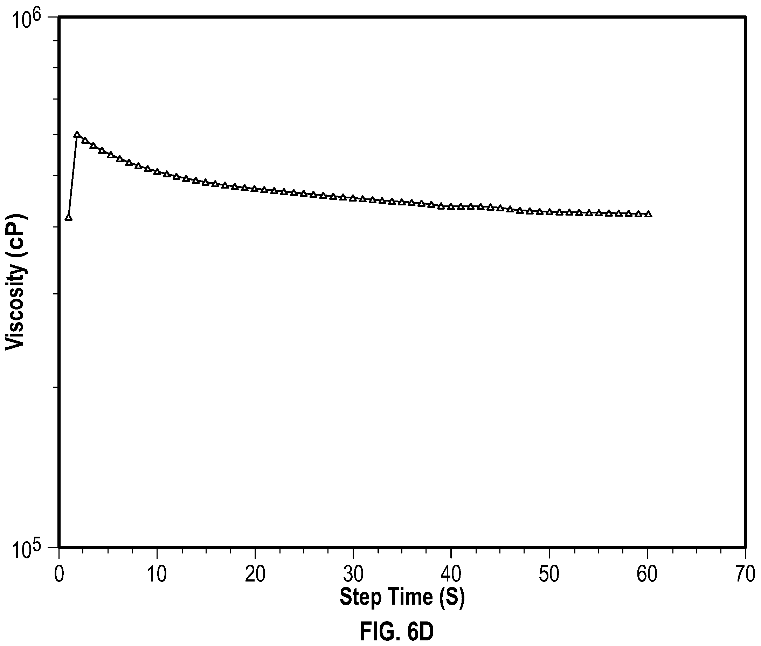

FIG. 6D is a graph illustrating the viscosity of the hydrogel. The viscosity was at least 400,000 cP (400 Pa-s) when measured at a shear rate of 1/sec. These results indicate that at low shear rates, the hydrogel exhibited high viscosity and behaved like a solid, thus providing sufficient cohesive strength to occlude a lesion.

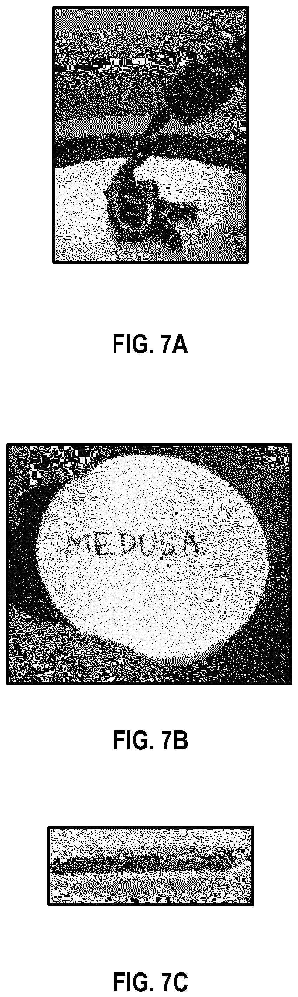

FIG. 7A is a photograph of the hydrogel being injected from a syringe. As shown in FIG. 7A, the hydrogel formed a cohesive cast after injection.

FIG. 7B is a photograph of the hydrogel after injection onto a surface. The hydrogel remained in place and did not flow when the injection stress was relieved.

FIG. 7C is a photograph illustrating occlusion of a simulated vessel with the hydrogel. The hydrogel was injected by hand through a 25-gauge needle and into a silicone vessel with a 2 mm inner diameter with a water flow rate of 10 mL/min. The hydrogel successfully occluded the vessel without leaking.

Overall, these results demonstrate that that a hydrogel composed of a synthetic anionic polyelectrolyte and silicate nanoparticles exhibits suitable properties for use as a liquid embolic system. When not subjected to shear stress, the electrostatic interactions between the particles and the polymer provided sufficient cohesive strength to occlude a vascular defect (e.g., the hydrogel behaves like a solid). When under shear stress, these interactions were disrupted and the anisotropy in the shape of the particles resulted in a large reduction in viscosity, which allows for injectability of the hydrogel.

### Additional Examples

Various examples of aspects of the present technology are described as numbered clauses (1, 2, 3, etc.) for convenience. These various aspects are provided as examples and do not limit the present technology.

Clause 1. A hydrogel composition for treating a vascular defect, the hydrogel composition comprising:
a plurality of silicate nanoparticles, wherein each silicate nanoparticle comprises a cationic portion and an anionic portion, and wherein the silicate nanoparticles are non-covalently crosslinked with each other via electrostatic interactions between the cationic portions and the anionic portions of the silicate nanoparticles; and
a synthetic polymer comprising an anionic functional group, wherein the synthetic polymer is non-covalently crosslinked with the plurality of silicate nanoparticles via electrostatic interactions between the anionic functional group of the synthetic polymer and the cationic portions of the silicate nanoparticles.

Clause 2. The hydrogel composition of Clause 1, wherein the synthetic polymer comprises one or more of the following: poly(acrylic acid), poly(methacrylic acid), poly(methyl methacrylate/methacrylic acid), oxidized poly(vinyl pyrrolidone), poly(ethylene glycol) diacid, carboxylic-acid functionalized poly(ethylene glycol) star polymer, poly(propylene glycol) diacid, poly(styrenesulfonic acid), poly(vinylsulfonic acid), poly(maleic acid), poly(butadiene/maleic acid), or poly(vinylphosphoric acid), or a salt thereof.

Clause 3. The hydrogel composition of Clause 1 or 2, wherein the anionic functional group is a carboxylic acid group.

Clause 4. The hydrogel composition of any one of Clauses 1 to 3, wherein the hydrogel composition comprises from 0.1% w/w to 5% w/w of the synthetic polymer.

Clause 5. The hydrogel composition of any one of Clauses 1 to 4, wherein the synthetic polymer has a viscosity average molecular weight within a range from 500 kDa to 2 MDa.

Clause 6. The hydrogel composition of any one of Clauses 1 to 5, wherein the silicate nanoparticles comprise one or more of the following: laponite, montmorillonite, bentonite, kaolinite, chlorite, illite, saponite, or hectorite.

Clause 7. The hydrogel composition of any one of Clauses 1 to 6, wherein the silicate nanoparticles are disk-shaped nanoparticles.

Clause 8. The hydrogel composition of Clause 7, wherein the cationic portions comprise disk edges of the disk-shaped nanoparticles and the anionic portions comprise disk faces of the disk-shaped nanoparticles.

Clause 9. The hydrogel composition of any one of Clauses 1 to 8, wherein the hydrogel composition comprises from 10% w/w to 20% w/w of the silicate nanoparticles.

Clause 10. The hydrogel composition of any one of Clauses 1 to 9, wherein the synthetic polymer comprises poly(acrylic acid) or poly(acrylic acid) sodium salt, and wherein the silicate nanoparticles comprise laponite.

Clause 11. The hydrogel composition of any one of Clauses 1 to 10, wherein the hydrogel composition is shear-thinning.

Clause 12. The hydrogel composition of Clause 11, wherein the hydrogel composition has a viscosity at 37 °C that is greater than 1000 Pa-s at a shear rate less than 10⁻² sec⁻¹ and that is less than 1 Pa-s at a shear rate greater than 10² sec⁻¹.

Clause 13. The hydrogel composition of Clause 11 or 12, wherein the hydrogel composition is configured to exhibit a first viscosity during injection of the hydrogel composition through a catheter, and a second viscosity after delivery of the hydrogel composition into the vascular defect, the second viscosity being higher than the first viscosity.

Clause 14. The hydrogel composition of any one of Clauses 1 to 13, further comprising a contrast agent.

Clause 15. The hydrogel composition of Clause 14, wherein the contrast agent comprises one or more of the following: tantalum, bismuth trioxide, bismnuth oxychloride, tungsten, tungsten carbide, barium sulfate, gadolinium, iodized oil, iohexol, iopamidol, ioxilan, iopromide, iodixanol, iobitridol, ioversol, diatrizoate, metrizoate, iothalamate, ioxaglate, iothalamate/meglumine, ioxaglate/meglumine, diatrizoate/meglumine, iodomide sodium, or metrizamide.

Clause 16. A method for treating a vascular defect, the method comprising:
delivering a hydrogel into the vascular defect to occlude the vascular defect, wherein the hydrogel comprises:
a plurality of silicate nanoparticles, wherein each silicate nanoparticle comprises a cationic portion and an anionic portion, and wherein the silicate nanoparticles are non-covalently crosslinked with each other via electrostatic interactions between the cationic portions and the anionic portions of the silicate nanoparticles, and
a synthetic polymer comprising an anionic functional group, wherein the synthetic polymer is non-covalently crosslinked with the plurality of silicate nanoparticles via electrostatic interactions between the anionic functional group of the synthetic polymer and the cationic portions of the silicate nanoparticles.

Clause 17. The method of Clause 16, wherein the synthetic polymer comprises one or more of the following: poly(acrylic acid), poly(methacrylic acid), poly(methyl methacrylate/methacrylic acid), oxidized poly(vinyl pyrrolidone), poly(ethylene glycol) diacid, carboxylic-acid functionalized poly(ethylene glycol) star polymer, poly(propylene glycol) diacid, poly(styrenesulfonic acid), poly(vinylsulfonic acid), poly(maleic acid), poly(butadiene/maleic acid), or poly(vinylphosphoric acid), or a salt thereof.

Clause 18. The method of Clause 16 or 17, wherein the anionic functional group is a carboxylic acid group.

Clause 19. The method of any one of Clauses 16 to 18, wherein the hydrogel comprises from 0.1% w/w to 5% w/w of the synthetic polymer.

Clause 20. The method of any one of Clauses 16 to 19, wherein the synthetic polymer has a viscosity average molecular weight within a range from 500 kDa to 2 MDa.

Clause 21. The method of any one of Clauses 16 to 20, wherein the silicate nanoparticles comprise one or more of the following: laponite, montmorillonite, bentonite, kaolinite, chlorite, illite, saponite, or hectorite.

Clause 22. The method of any one of Clauses 16 to 21, wherein the silicate nanoparticles are disk-shaped nanoparticles.

Clause 23. The method of Clause 22, wherein the cationic portions comprise disk edges of the disk-shaped nanoparticles and the anionic portions comprise disk faces of the disk-shaped nanoparticles.

Clause 24. The method of any one of Clauses 16 to 23, wherein the hydrogel comprises from 10% w/w to 20% w/w of the silicate nanoparticles.

Clause 25. The method of any one of Clauses 16 to 24, wherein the synthetic polymer comprises poly(acrylic acid) or poly(acrylic acid) sodium salt, and wherein the silicate nanoparticles comprise laponite.

Clause 26. The method of any one of Clauses 16 to 25, wherein the hydrogel is shear-thinning.

Clause 27. The method of Clause 26, wherein the hydrogel has a viscosity at 37 °C that is greater than 1000 Pa-s at a shear rate less than 10⁻² sec⁻¹ and that is less than 1 Pa-s at a shear rate greater than 10² sec⁻¹.

Clause 28. The method of any one of Clauses 16 to 27, wherein the hydrogel is delivered into the vascular defect via injection through a catheter.

Clause 29. The method of Clause 28, wherein the hydrogel has a first viscosity during the injection of the hydrogel through the catheter, and a second viscosity after delivery of the hydrogel into the vascular defect, the second viscosity being higher than the first viscosity.

Clause 30. The method of any one of Clauses 16 to 29, wherein the hydrogel further comprises a contrast agent.

Clause 31. The method of Clause 30, wherein the contrast agent comprises one or more of the following: tantalum, bismuth trioxide, bismuth oxychloride, tungsten, tungsten carbide, barium sulfate, gadolinium, iodized oil, iohexol, iopamidol, ioxilan, iopromide, iodixanol, iobitridol, ioversol, diatrizoate, metrizoate, iothalamate, ioxaglate, iothalamate/meglumine, ioxaglate/meglumine, diatrizoate/meglumine, iodomide sodium, or metrizamide.

Clause 32. The method of any one of Clauses 16 to 31, further comprising positioning a neck cover within the vascular defect before delivering the hydrogel into the vascular defect.

### Conclusion

Although many of the embodiments are described above with respect to systems, devices, and methods for treating saccular intracranial aneurysms, the technology is applicable to other applications and/or other approaches. For example, suitable features of described systems, devices, compositions, and methods for treating saccular intracranial aneurysms can be implemented in the context of treating non-saccular intracranial aneurysms, abdominal aortic aneurysms, thoracic aortic aneurysms, renal artery aneurysms, arteriovenous malformations (e.g., brain arteriovenous malformations), arteriovenous fistulas, tumors (e.g., via occlusion of vessel(s) feeding a tumor), chronic subdural hematomas (e.g., via occlusion of the middle meningeal artery), perivascular leaks, varicose veins (e.g., via occlusion of one or more truncal veins such as the great saphenous vein), hemorrhoids, and sealing endoleaks adjacent to artificial heart valves, covered stents, and abdominal aortic aneurysm devices, among other examples. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described above with reference to FIGS. 1A-7C.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. As used herein, the phrase "and/or" as in "A and/or B" refers to A alone, B alone, and A and B. Additionally, the term "comprising" is used throughout to mean including at least the recited feature(s) such that any greater number of the same feature and/or additional types of other features are not precluded.

To the extent any materials incorporated herein by reference conflict with the present disclosure, the present disclosure controls.

It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A hydrogel composition for use in treating a vascular defect, the hydrogel composition comprising:
a plurality of silicate nanoparticles, wherein each silicate nanoparticle comprises a cationic portion and an anionic portion, and wherein the silicate nanoparticles are non-covalently crosslinked with each other via electrostatic interactions between the cationic portions and the anionic portions of the silicate nanoparticles; and
a synthetic polymer comprising an anionic functional group, wherein the synthetic polymer is non-covalently crosslinked with the plurality of silicate nanoparticles via electrostatic interactions between the anionic functional group of the synthetic polymer and the cationic portions of the silicate nanoparticles.

2. The hydrogel composition of claim 1, wherein the synthetic polymer comprises one or more of the following: poly(acrylic acid), poly(methacrylic acid), poly(methyl methacrylate/methacrylic acid), oxidized poly(vinyl pyrrolidone), poly(ethylene glycol) diacid, carboxylic-acid functionalized poly(ethylene glycol) star polymer, poly(propylene glycol) diacid, poly(styrenesulfonic acid), poly(vinylsulfonic acid), poly(maleic acid), poly(butadiene/maleic acid), or poly(vinylphosphoric acid), or a salt thereof.

3. The hydrogel composition of claim 1 or 2, wherein the anionic functional group is a carboxylic acid group.

4. The hydrogel composition of any preceding claim, wherein the hydrogel composition comprises from 0.1% w/w to 5% w/w of the synthetic polymer.

5. The hydrogel composition of any preceding claim, wherein the synthetic polymer has a viscosity average molecular weight within a range from 500 kDa to 2 MDa.

6. The hydrogel composition of any preceding claim, wherein the silicate nanoparticles comprise one or more of the following: laponite, montmorillonite, bentonite, kaolinite, chlorite, illite, saponite, or hectorite.

7. The hydrogel composition of any preceding claim, wherein the silicate nanoparticles are disk-shaped nanoparticles.

8. The hydrogel composition of claim 7, wherein the cationic portions comprise disk edges of the disk-shaped nanoparticles and the anionic portions comprise disk faces of the disk-shaped nanoparticles.

9. The hydrogel composition of any preceding claim, wherein the hydrogel composition comprises from 10% w/w to 20% w/w of the silicate nanoparticles.

10. The hydrogel composition of any preceding claim, wherein the synthetic polymer comprises poly(acrylic acid) or poly(acrylic acid) sodium salt, and wherein the silicate nanoparticles comprise laponite.

11. The hydrogel composition of any preceding claim, wherein the hydrogel composition is shear-thinning.

12. The hydrogel composition of claim 11, wherein the hydrogel composition has a viscosity at 37 °C that is greater than 1000 Pa-s at a shear rate less than 10⁻² sec⁻¹ and that is less than 1 Pa-s at a shear rate greater than 10² sec⁻¹.

13. The hydrogel composition of claim 11 or 12, wherein the hydrogel composition is configured to exhibit a first viscosity during injection of the hydrogel composition through a catheter, and a second viscosity after delivery of the hydrogel composition into the vascular defect, the second viscosity being higher than the first viscosity.

14. The hydrogel composition of any preceding claim, further comprising a contrast agent, optionally wherein the contrast agent comprises one or more of the following: tantalum, bismuth trioxide, bismnuth oxychloride, tungsten, tungsten carbide, barium sulfate, gadolinium, iodized oil, iohexol, iopamidol, ioxilan, iopromide, iodixanol, iobitridol, ioversol, diatrizoate, metrizoate, iothalamate, ioxaglate, iothalamate/meglumine, ioxaglate/meglumine, diatrizoate/meglumine, iodomide sodium, or metrizamide.

15. The hydrogel composition of any preceding claim, wherein said hydrogel composition is to be delivered into the vascular defect to occlude the vascular defect.
